# FASCICULE DE BREVET EUROPEEN

(11) **EP 2 307 438 B1**
(45) Date de publication et mention de la délivrance du brevet: **07.03.2018**
(21) Numéro de dépôt: 09784316.3
(22) Date de dépôt: 29.07.2009
(51) Int. Cl.: C07J 41/00, C07J 43/00, A61K 31/58, A61K 31/575, A61P 25/28

(54) **DÉRIVÉS DE L'OXIME DE CHOLEST-4-ÈN-3-ONE, COMPOSITIONS PHARMACEUTIQUES LES RENFERMANT, ET PROCÉDÉ DE PRÉPARATION**
CHOLEST-4-EN-3-ON OXIMDERIVATE, PHARMAZEUTISCHE ZUSAMMENSETZUNGEN, DIE DIESE ENTHALTEN, UND HERSTELLUNGSVERFAHREN
CHOLEST-4-EN-3-ONE OXIME DERIVATIVES, PHARMACEUTICAL COMPOSITIONS CONTAINING SAME, AND PREPARATION METHOD

(30) Priorité: 30.07.2008 FR 0804338
(43) Date de publication de la demande: 13.04.2011
(73) Titulaire: Trophos, 13288 Marseille Cedex 9 (FR)
(72) Inventeur: PRUSS, Rebecca, F-13260 Cassis (FR); NAZIH, Abdesslame, F-13008 Marseille (FR); CHAIMBAULT, Corinne, F-13009 Marseille (FR); DROUOT, Cyrille, F-83300 Draguignan (FR)
(74) Mandataire: Santarelli
(86) Numéro de dépôt international: PCT/FR2009/000944
(87) Numéro de publication internationale: WO 2010/012904

(56) Documents cités:
- WO-A-2004/082581
- FR-A- 2 894 968
- HAUPTMANN, HAGEN ET AL: "Concepts for the syntheses of biotinylated steroids. Part I: Testosterone derivatives as immunochemical probes" BIOCONJUGATE CHEMISTRY, vol. 11, no. 2, 20 mars 2000 (2000-03-20), pages 239-252, XP002567556 ISSN: 1043-1802 DOI: 10.1021/bc9901402
- TEUTSCH G ET AL: "Synthesis of a fluorescent steroid derivative with high affinities for the glucocorticoid and progesterone receptors" STEROIDS, ELSEVIER SCIENCE PUBLISHERS, NEW YORK, NY, US, vol. 59, no. 1, 1 janvier 1994 (1994-01-01), pages 22-26, XP025217340 ISSN: 0039-128X DOI: 10.1016/0039-128X(94)90040-X [extrait le 1994-01-01]

## Description

La présente invention concerne de nouveaux composés chimiques, particulièrement des dérivés d'oxime de cholest-4-èn-3-one, leur application à titre de médicaments, notamment comme médicaments cytoprotecteurs, particulièrement comme médicaments neuroprotecteurs, cardioprotecteurs et/ou hépatoprotecteurs.

Lesdits médicaments sont particulièrement adaptés aux pathologies et aux traumatismes liés à la dégénérescence ou à la mort cellulaire, particulièrement celles des motoneurones et/ou des cardiomyocytes et/ou des hépatocytes.

L'invention concerne également les compositions pharmaceutiques renfermant lesdits composés, et leur procédé de préparation.

Les processus dégénératifs cellulaires sont caractérisés par le dysfonctionnement des cellules entraînant souvent des activités cellulaires indésirables et la mort cellulaire.

Les cellules ont développé des mécanismes d'adaptation, en réponse au stress, qui allongent leur durée de vie ou retardent ou empêchent la mort cellulaire (mécanismes cytoprotecteurs).

Cependant, ces mécanismes cytoprotecteurs sont parfois insuffisants, inadéquats, ou induits trop tard pour être efficaces et les cellules meurent. Il peut donc s'avérer intéressant de disposer de nouveaux médicaments, cytoprotecteurs, qui favoriseraient la cytoprotection.

Parmi les mécanismes principaux de mort cellulaire, on distingue essentiellement la nécrose, l'apoptose et la nécroptose.

La nécrose est une mort cellulaire dite "accidentelle" qui survient lors d'un dommage tissulaire. C'est la membrane plasmique de la cellule qui est la plus touchée, entraînant une modification de l'homéostasie de la cellule. Les cellules vont se gorger d'eau au point que cela va entraîner la lyse de leur membrane plasmique. Cette lyse cellulaire conduit au largage dans le milieu environnant du contenu cytoplasmique. La nécrose est à l'origine du processus inflammatoire.

La nécrose peut toucher un ensemble de cellules ou un tissu alors que les autres parties de voisinage restent vivantes. La transformation qui en résulte est une mortification des cellules ou des tissus.

Autrement dit, la nécrose se définit par des modifications morphologiques survenant lorsqu'une cellule arrive en fin de vie à la suite d'événements tels qu'un traumatisme important comme un arrêt ou une diminution de la circulation sanguine au niveau d'un organe, l'hyperthermie (élévation importante de la température), une intoxication par un produit chimique, un choc physique, etc...

Une des nécroses les plus connues est celle du myocarde lors de l'infarctus (arrêt d'apport circulatoire au niveau du muscle cardiaque) due à une oblitération (obstruction) d'une artère coronaire.

L'apoptose fait partie intégrante de la physiologie normale d'un organisme. C'est une forme physiologique de mort cellulaire hautement régulée et elle est nécessaire à la survie des organismes multicellulaires. L'apoptose est un processus qui joue un rôle primordial au cours de l'embryogenèse.

Les cellules en apoptose ou apoptotiques vont s'isoler des autres cellules. L'apoptose implique habituellement des cellules individuelles dans un tissu et ne provoque pas l'inflammation. L'un des points morphologiques caractéristiques de l'apoptose est l'importante condensation à la fois du noyau et du cytoplasme ce qui induit une diminution significative du volume cellulaire. Le noyau se fragmente ensuite, chaque fragment est entouré d'une double enveloppe. Des corps apoptotiques (éléments cytoplasmiques et nucléaires) sont ensuite libérés et vont être absorbés par phagocytose par les cellules voisines.

L'apoptose peut être induite de différentes façons. Par exemple, une radiation, la présence d'un composé chimique ou d'une hormone sont des stimuli susceptibles d'induire une cascade d'événements apoptotiques dans la cellule. Des signaux intracellulaires comme une mitose incomplète ou un dommage à l'ADN peuvent aussi induire l'apoptose.

L'apoptose intervient aussi après l'action d'un génotoxique ou au cours d'une maladie. Certaines pathologies sont caractérisées par une apoptose anormale, entraînant la perte de certaines populations cellulaires, comme par exemple l'hépatotoxicité, les rétinopathies, la cardiotoxicité.

On distingue donc l'apoptose physiologique et l'apoptose pathologique. L'invention s'adresse essentiellement à l'apoptose pathologique.

Il existe d'autres mécanismes de mort cellulaire, comme par exemple la nécroptose, qui présente des caractéristiques de la nécrose et de l'apoptose. Une cellule mourant par nécroptose présente des caractéristiques similaires à celles d'une cellule mourant par nécrose, mais les étapes biochimiques de ce mécanisme s'assimilent plus à celles de l'apoptose. Ce mécanisme de mort cellulaire intervient par exemple dans l'ischémie.

C'est donc aussi un des buts de la présente invention que de disposer de nouveaux médicaments qui pourraient permettre de prévenir et/ou traiter la nécrose et/ou l'apoptose pathologique et/ou la nécroptose (médicaments antinécrotiques et/ou antiapoptotiques et/ou antinécroptotiques).

Les processus dégénératifs cellulaires peuvent résulter, entre autres, de situations pathologiques regroupées sous le terme de maladies ou affections dégénératives, de traumatismes, ou d'exposition à divers facteurs.

Ces traumatismes et facteurs peuvent inclure, par exemple, l'exposition aux radiations (UV, gamma), l'hypoxie ou la privation d'oxygène, la privation de nutriments, la privation de facteurs de croissance, des poisons, des toxines cellulaires, des déchets, des toxines environnementales, des radicaux libres, des oxygènes réactifs. On peut citer également des agents chimiques ou biologiques utilisés comme agents thérapeutiques dans le contexte de traitements médicaux comme par exemple des agents cytostatiques ou des agents anti-inflammatoires.

Parmi les situations pathologiques caractérisées par un processus dégénératif les plus importantes, on trouve :
▪ les maladies des os, des articulations, du tissu conjonctif et du cartilage, telles que l'ostéoporose, l'ostéomyélite, les arthrites dont par exemple l'ostéoarthrite, l'arthrite rhumatoïde et l'arthrite psoriasique, la nécrose avasculaire, la fibrodysplasie ossifiante progressive, le rachitisme, le syndrome de Cushing ;
▪ les maladies musculaires telles que la dystrophie musculaire, comme par exemple la dystrophie musculaire de Duchenne, les dystrophies myotoniques, les myopathies et les myasthénies ;
▪ les maladies de la peau, telles que les dermatites, l'eczéma, le psoriasis, le vieillissement, ou encore les altérations de la cicatrisation ;
▪ les maladies cardiovasculaires telles que l'ischémie cardiaque et/ou vasculaire, l'infarctus du myocarde, la cardiopathie ischémique, l'insuffisance cardiaque chronique ou aigue, la dysrythmie cardiaque, la fibrillation auriculaire, la fibrillation ventriculaire, la tachycardie paroxystique, l'insuffisance cardiaque, l'anoxie, l'hypoxie, les effets secondaires dus à des thérapies avec des agents anticancéreux ;
▪ les maladies circulatoires telles que l'athérosclérose, les scléroses artérielles, les maladies vasculaires périphériques, les accidents vasculaires cérébraux, les anévrismes ;
▪ les maladies hématologiques et vasculaires telles que : l'anémie, l'amyloïdose vasculaire, les hémorragies, la drépanocytose, le syndrome de la fragmentation des globules rouges, la neutropénie, la leucopénie, l'aplasie médullaire, la pancytopénie, la thrombocytopénie, l'hémophilie ;
▪ les maladies du poumon incluant la pneumonie, l'asthme ; les maladies chroniques obstructives des poumons comme par exemple les bronchites chroniques et l'emphysème ;
▪ les maladies du tractus gastro-intestinal, telles que les ulcères ;
▪ les maladies du foie incluant les hépatites virales et les cirrhoses, les maladies du foie dues à des toxines ou à des médicaments, les affections susceptibles d'évoluer vers une cirrhose telles que la stéato-hépatite non alcoolique (Non Alcoholic SteatoHepatitis, NASH), la maladie de Wilson, la cholangite sclérosante primitive, ou la cirrhose biliaire primitive
▪ les maladies du pancréas comme par exemple les pancréatites aigues ou chroniques ;
▪ les maladies métaboliques telles que les diabètes mellitus et insipide, les thyroïdites ;
▪ les maladies des reins telles que par exemple les désordres rénaux aigus ou la glomérulonéphrite ;
▪ les infections virales et bactériennes telle que la septicémie ;
▪ les intoxications sévères par des agents chimiques, des toxines ou des médicaments ;
▪ les affections dégénératives associées au Syndrome Immuno Déficitaire Acquis (SIDA) ;
▪ les désordres associés au vieillissement, tel que le syndrome du vieillissement accéléré ;
▪ les maladies inflammatoires, telles que la maladie de Crohn, la polyarthrite rhumatoïde ;
▪ les maladies auto-immunes telles que le lupus érythémateux ;
▪ les désordres dentaires tels que ceux aboutissant à la dégradation des tissus comme par exemple les périodontites ;
▪ les maladies ou désordres ophtalmiques incluant les rétinopathies diabétiques, le glaucome, les dégénérescences maculaires, la dégénérescence rétinienne, la rétinite pigmentaire, les trous ou déchirures rétiniennes, le décollement rétinien, l'ischémie rétinienne, les rétinopathies aigues associées à un traumatisme, les dégénérescences inflammatoires, les complications post chirurgicales, les rétinopathies médicamenteuses, la cataracte ;
▪ les désordres des voies auditives, tels que l'otosclérose et la surdité induite par des antibiotiques ;
▪ les maladies associées aux mitochondries (pathologies mitochondriales), telles que l'ataxie de Friedrich, la dystrophie musculaire congénitale avec anomalie mitochondriale structurelle, certaines myopathies (syndrome des MELAS, syndrome de MERFF, syndrome de Pearson), le syndrome de MIDD (diabètes mitochondriaux et surdité), le syndrome de Wolfram, la dystonie.

Par ailleurs, les processus neurodégénératifs sont caractérisés par le dysfonctionnement et la mort des neurones entraînant la perte des fonctions neurologiques médiées par le cerveau (système nerveux central, SNC), la moelle épinière et le système nerveux périphérique (SNP). Ils peuvent résulter, entre autres, de situations pathologiques regroupées sous le terme de maladies ou affections neurodégénératives, de traumatisme, ou d'exposition à des toxines.

Les pathologies les plus importantes qui sont caractérisées par un processus neurodégénératif sont :
- les maladies chroniques neurodégénératives, notamment les maladies chroniques démyélinisantes , héréditaires ou sporadiques, avantageusement la maladie d'Alzheimer, la maladie de Huntington, la maladie de Parkinson, la sclérose latérale amyotrophique, les amyotrophies spinales, particulièrement infantiles, la maladie de Creutzfeldt-Jakob, la sclérose en plaque, les scléroses latérales amyotrophiques, les leucodystrophies dont l'adrénoleucodystrophie, l'épilepsie, les démences, la schizophrénie, et les syndromes neurologiques associés au SIDA;
- les lésions neuronales liées au vieillissement ;
- les neuropathies périphériques héréditaires ou lésionnelles, comme les maladies de Fabry, de Charcot-Marie-Tooth, de Krabbe, les leucodystrophies, les neuropathies diabétiques et celles induites par les traitements anti-cancéreux ;
- les traumatismes du cerveau, des nerfs périphériques ou de la moelle épinière;
- les ischémies du cerveau ou de la moelle épinière suite à un accident cérébro-vasculaire, ou induites par un manque d'irrigation sanguine ;
- les dégénérescences, héréditaires, lésionnelles ou liées au vieillissement des neurones sensoriels de la vision, comme les dégénérescences maculaires, les rétinites pigmentaires, ou les dégénérescences du nerf optique induites par les glaucomes ;
- les dégénérescences, héréditaires, traumatiques ou liées au vieillissement des neurones sensoriels de l'ouïe entraînant une diminution ou une perte de l'audition.

Une partie des voies de signalisation affectées dans ces pathologies sont communes à un grand nombre de maladies neurodégénératives. La maladie d'Alzheimer est la démence la plus fréquente. Elle fait apparaître une atrophie du cerveau, une perte neuronale prédominante dans la corne d'Ammon et elle touche aussi les neurones cholinergiques. D'autres pathologies, comme les atrophies lobaires (maladie de Pick, la maladie de Creutzfeld-Jakob), la démence avec corps de Lewy, les démences vasculaires, la maladie de Parkinson sont associées à une mort neuronale importante à l'origine des symptômes de ces démences.

Une approche thérapeutique pour protéger les neurones de la mort est l'apport de protéines neurotrophiques.

Ces protéines, telles que BDNF (brain-derived neurotrophic factor), CNTF (ciliary neurotrophic factor), NGF (nerve growth factor), GDNF (glia-derived neurotrophic factor) sont synthétisées au cours du développement embryonnaire ou après lésion chez l'adulte. Ces facteurs de croissance favorisent la survie, la maturation et la différentiation des cellules neuronales. De plus, ils inhibent les mécanismes apoptotiques, activent de multiples voies de survie et protègent un grand nombre de populations neuronales. Leur utilisation est proposée dans la plupart des dégénérescences neuronales.

Des composés qui activeraient l'expression de facteurs neurotrophiques ou qui mimeraient l'action de ces facteurs ont un potentiel thérapeutique pour le traitement des syndromes neurodégénératifs.

En particulier, l'apport de molécules neurotrophiques pour le traitement des dégénérescences neuronales vise trois objectifs :
- compenser une carence potentielle en facteurs neurotrophiques liée à un défaut d'apport par les cibles périphériques ou centrales des neurones et/ou un trouble du transport rétrograde de ces facteurs ;
- intervenir de façon non spécifique sur des voies biochimiques impliquées dans la cascade dégénérative;
- favoriser les phénomènes compensateurs naturels de croissance dendritique et d'arborisation des terminaisons nerveuses.

Ces composés présenteraient donc un effet bénéfique dans un grand nombre de pathologies en particulier dans les pathologies touchant les systèmes nerveux périphérique et central.

Par ailleurs, dans le cadre ci-dessus, les motoneurones sont des neurones notamment présents dans la moelle épinière et le tronc cérébral. Leur dégénérescence ou leur mort peut conduire à une faiblesse progressive des muscles des membres, puis à une atrophie et éventuellement à une spasticité (c'est à dire une contraction permanente) du muscle.

Les pathologies les plus importantes qui résultent de la dégénérescence et de la mort des motoneurones spinaux et/ou bulbaires sont la sclérose latérale amyotrophique, également connue sous le nom de maladie de Charcot ou encore maladie de Lou Gehrig, et les amyotrophies spinales, particulièrement infantiles, également connues sous les noms de maladie de Werdnig-Hoffmann ou maladie de Kugelberg-Welander.

En outre, on observe une dégénérescence des motoneurones dans les cas de traumatismes avec écrasement et/ou section de la moelle épinière ou des nerfs moteurs périphériques.

Plus généralement, on parle d'amyotrophies spinales pour les maladies où est impliquée la dégénérescence ou la mort des motoneurones de la moelle épinière.

La sclérose latérale amyotrophique (SLA ou ALS pour Amyotrophic Lateral Sclerosis) est une maladie neurodégénérative associée à différents types d'inclusions tels les corps de Lewis et caractérisée par une dégénérescence des motoneurones spinaux et corticaux dont l'issue fatale est parfois associée à une démence frontale. Au cours du développement de l'ALS, les phénomènes dégénératifs se produisent non seulement dans le cerveau mais également dans la moelle épinière et en conséquence dans le muscle, par défaut d'innervation.

Sur le plan des structures chimiques, la littérature fournit quelques exemples de dérivés du 3-oxyimino-cholest-4-ène. C'est le cas notamment des demandes de brevet WO2004/082581 et WO2007/118967 décrivant le 3-oxyimino-cholest-4-ène et de ses dérivés pour leurs propriétés neuroprotectrices et cytoprotectrices respectivement.

Le document Liebigs Annalen der Chemie [(1991), (1), pages 89-91], décrit les composés 3-oxyimino-4-chloro-cholest-4-ène et 3-oxyimino-4-bromo-cholest-4-ène ainsi que leur propriété chiroptique. Mais ce document ne décrit aucune activité cytoprotectrice, neuroprotectrice et/ou cardioprotectrice pour ces deux composés.

Le document Chemical & Pharmaceutical Bulletin [(1972), 20(7), pages 1567-9], décrit l'isomère anti du 3-oxyimino-4-méthyl-cholest-4-ène, sans toutefois décrire ou évoquer d'éventuelles activités cytoprotectrices, neuroprotectrices, et/ou cardioprotectrices pour ce composé.

Sans toutefois dénigrer les traitements connus actuellement, il n'existe pas à ce jour de traitement efficace pour enrayer les dégénérescences cellulaires, particulièrement les dégénérescences neuronales.

Ainsi, il existe toujours un réel besoin de nouveaux produits permettant de protéger efficacement les cellules contre les phénomènes de dégénérescence.

Les composés de la présente invention, outre le fait qu'ils soient nouveaux, présentent des propriétés pharmacologiques très intéressantes.

Ils se révèlent avantageusement cytoprotecteurs, particulièrement neuroprotecteurs et/ou cardioprotecteurs et/ou hépatoprotecteurs.

En plus, de leur activité biologique, certains de ces nouveaux composés peuvent également présenter des propriétés avantageuses en relation avec leur activité pharmacologique, telle que leur pharmacocinétique, leur biodisponibilité, leur solubilité, leur stabilité, leur toxicité, leur absorption et/ou leur métabolisme. Ceci les rend très utiles pour la préparation d'un médicament, particulièrement pour la préparation d'un médicament cytoprotecteur, très particulièrement neuroprotecteur et/ou cardioprotecteur et/ou hépatoprotecteur.

Plus spécifiquement, la présente invention a pour objet les composés nouveaux suivant :
- le 3-oxyimino-4-fluoro-cholest-4-ène ;
- le 3-oxyimino-6β-fluoro-cholest-4-ène ;
- le 3-oxyimino-2,2-difluoro-cholest-4-ène ;
- le 3-oxyimino-2,6-difluoro-cholest-4-ène ;
- le 3-oxyimino-2α-fluoro-cholest-4-ène ;
- le 3-oxyimino-25-[méthyl(7-nitro-2,1,3-benzoxadiazol-4-yl)amino]-27-norcholest-4-ène ;
- le 3-oxyimino-25-((N-(+)-biotinoyl-N-méthyl) amino)-27-norcholest-4-ène ;
- le 3-oxyimino-19-hydroxy-cholest-4-ène ;
- le 3-oxyimino-19-biotinyloxy-cholest-4-ène ;
- le 3-oxyimino-2-méthyl-cholest-4-ène ;
- le 3-oxyimino-4-méthoxy-cholest-4-ène ;
ainsi que :
- leurs isomères *SYN*, *ANTI*, lorsqu'ils existent,
- leurs isomères optiques (énantiomères, diastéréoisomères), lorsqu'ils existent,
- leurs sels d'addition à un acide ou à une base pharmaceutiquement acceptable,
- leurs hydrates et leurs solvates,
- leurs prodrogues
Il est entendu que selon le présent texte,
➢ le terme "alkyle en C₁-C₆" fait référence à un radical hydrocarboné linéaire ou ramifié, comprenant de 1 à 6 atomes de carbone, tel que méthyle, éthyle, propyle, isopropyle, butyle, isobutyle, tert-butyle, pentyle, néopentyle, n-hexyle. Les groupes alkyles en C₁-C₄ sont préférés. Les groupes alkyles peuvent éventuellement être substitués par un groupe aryle tel que défini ci-après, auquel cas on parle de groupe arylalkyle. Des exemples de groupes arylalkyle sont notamment benzyle et phénéthyle. Optionnellement, les groupes alkyles peuvent être substitués une ou plusieurs fois par un des substituants choisis indépendamment parmi un atome d'halogène ou un groupe -CN, -CF₃, -COOR^{a}, -CONR^{a}R^{b}, -O-CONR^{a}R^{b}, -NR^{a}R^{b}, -OR^{a}, -SR^{b}, les groupes R^{a} et R^{b} étant tels que décrits précédemment. Sauf dans les cas où il est spécifié, le radical hydrocarboné peut comporter de 4 à 12 atomes de carbones. Le terme "alcényle en C₂-C₆" fait référence à un radical hydrocarboné linéaire ou ramifié ou cyclique, comprenant une ou plusieurs double-liaisons, ayant de 2 à 6 atomes de carbone. On peut citer, par exemple, le radical éthényle, 1-propényle, 2-propényle, 1-butényle, 2-butényle, 1-pentényle, 2-pentényle, 3-méthyl-3-butényle, 1-hexényle. Optionnellement, les groupes alcényles peuvent être substitués une ou plusieurs fois par un des substituants choisis indépendamment parmi un atome d'halogène ou un groupe -CN, -CF₃, -COOR^{a},-C(O)NR^{a}R^{b}, -O-C(O)NR^{a}R^{b}, -NR^{a} R^{b}, -OR^{a}, -SR^{b}, les groupes R^{a}, R^{b} étant tels que décrits précédemment ;
➢ le terme "cycloalkyle en C₃-C₆" fait référence à un radical hydrocarboné cyclique saturé ou partiellement insaturé, ayant de 3 à 6 atomes de carbone. Les groupes cycloalkyles incluent notamment les substituants cyclopropyle, cyclobutyle, cyclopentyle, cyclopéntyle, cyclohexyle, cyclohéxényle. Optionnellement, les groupes cycloalkyles peuvent être substitués une ou plusieurs fois par un des substituants choisis indépendamment parmi un atome d'halogène ou un groupe -CN, -CF₃, -COOR^{a}, -C(O)NR^{a}R^{b}, -O-C(O)NR^{a}R^{b}, -NR^{a}R^{b}, -OR^{a}, -SR^{a}, le groupe R^{a}, R^{b} étant tels que décrits précédemment ;
➢ le terme "alcynyle en C₂-C₆" fait référence à un radical hydrocarboné linéaire, ramifié comprenant au moins une triple liaison, ayant de 2 à 6 atomes de carbone. Les groupes alcynyles incluent notamment les susbtituants éthynyle, 1-propynyle, 2-propynyle, 1-butynyle, 2-butynyle, 1-pentynyle ou 2-pentynyle. Optionnellement, les groupes alcynyles peuvent être substitués une ou plusieurs fois par un des substituants choisis indépendamment parmi un atome d'halogène ou un groupe -CN, -CF₃, -COOR^{a}, -C(O)NR^{a}R^{b}, -O-C(O)NR^{a}R^{b}, -NR^{a}R^{b}, -OR^{a}, -SR^{a}, le groupe R^{a}, R^{b} étant tels que décrits précédemment ;
➢ le terme "aryle en C₆-C₁₀" fait référence à un radical hydrocarboné aromatique, ayant 6 à 10 atomes de carbone, encore plus préférentiellement 6 atomes de carbone. Les groupes aryles incluent notamment les radicaux phényle, naphtyle et bi-phényle. Optionnellement, les groupes aryles peuvent être substitués une ou plusieurs fois par un des substituants choisis indépendamment parmi un atome d'halogène ou un groupe alkyle, -CN, -CF₃, -N₃, -NO₂, -COOR^{a}, -C(O)NR^{a}R^{b}, -O-C(O)NR^{a}R^{b}, -NR^{a}R^{b}, -OR^{a}, -SR^{a}, les groupes R^{a}, R^{b} étant tels que décrits précédemment ;
➢ le terme "hétérocycle en C₃-C₉" fait référence à un radical mono- ou polycyclique, saturé, insaturé ou aromatique, éventuellement substitué, comportant de 3 à 9 atomes de carbone et comprenant un ou plusieurs hétéroatomes. De préférence, les hétéroatomes sont choisis parmi l'oxygène, le soufre et l'azote. Des exemples d'hétérocycle sont les radicaux furyle, thiényle, pyrrole, imidazole, isothiazole, thiazole, isoxazole, oxazole, pyridine, pyrazine, pyrimidine, pyridazine, indole, isoindole, indazole, quinoléine, isoquinoléine, phthalazine, quinazoline, pyrrolidine, imidazolidine, pyrrazolidine, pipéridine, pipérazine, morpholine, thiazolidine ou phthalimide. benzimidazole. Optionnellement, les groupes hétérocyles peuvent être substitués une ou plusieurs fois par un des substituants choisis indépendamment parmi un atome d'halogène ou un groupe alkyle, -CN, -CF₃, -N₃, -NO₂, -COOR^{a}, -C(O)NR^{a}R^{b}, -O-C(O)NR^{a}R^{b}, -NR^{a}R^{b}, -OR^{a}, -SR^{a}, les groupes R^{a}, R^{b} étant tels que décrits précédemment ;
➢ le terme "halogène" fait référence à un atome de chlore, brome, fluor et iode. De préférence un atome de fluor ;
➢ l'expression "bras espaceur" fait référence à une chaîne hydrocarbonée comportant de 2 à 20 atomes de carbone, saturée ou non, éventuellement substituée et comprenant au moins un hétéroatome. En particulier ledit bras espaceur est choisi parmi les groupes K1, K2, K3 ou K4 suivant dans lesquels R^{a} est tel que décrit précédemment : 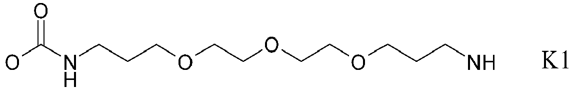 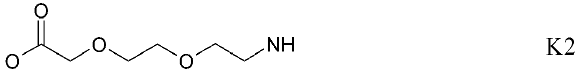 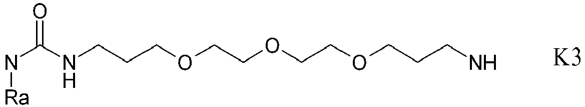 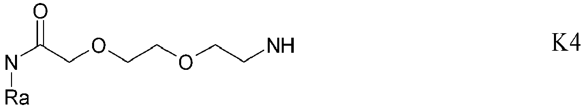
➢ le terme "traitement" désigne le traitement préventif, curatif, palliatif, ainsi que la prise en charge des patients (réduction de la souffrance, amélioration de la durée de vie, ralentissement de la progression de la maladie), etc. Le traitement peut en outre être réalisé en combinaison avec d'autres ingrédients ou traitements, tels que notamment d'autres composés actifs pour traiter les pathologies ou traumatismes spécifiés dans la présente demande ;
➢ le terme "cytoprotecteur" fait référence à la capacité d'agents, par exemple des composés chimiques, naturels ou non, à maintenir les interactions des cellules entre elles ou avec les autres tissus, à protéger les cellules contre les phénomènes de dégénérescence conduisant à une perte de fonction cellulaire ou à des activités cellulaires indésirables, avec ou sans mort cellulaire, et/ou contre les dysfonctionnements cellulaires et/ou contre les maladies ou affections dégénératives conduisant à ces dysfonctionnements cellulaires, lesdits dysfonctionnements ou lesdites maladies ou affections conduisant ou non à la mort cellulaire ;
➢ les termes "neuroprotecteur" ou "cardioprotecteur" ou "hépatoprotecteur" font référence aux mêmes propriétés desdits agents mais spécifiquement pour les cellules du système nerveux ("neuroprotecteur") soit spécifiquement pour les cellules du système cardiaque ("cardioprotecteur"), soit spécifiquement pour les cellules du système hépatique ("hépatoprotecteur"). On comprend donc qu'un composé cytoprotecteur ou neuroprotecteur ou cardioprotecteur ou hépatoprotecteur est un composé qui présente les propriétés précédemment décrites.

Les sels d'addition avec les acides pharmaceutiquement acceptables peuvent être par exemple des sels formés avec les acides chlorhydrique, bromhydrique, nitrique, sulfurique, phosphorique acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques, ou carboxyliques.

Certains composés préférés de la présente invention possèdent un ou plusieurs atomes de fluor. A titre d'exemple, le 3-oxyimino-4-fluoro-cholest-4-ène (Formule I-1) possédant un atome de fluor en position 4 est particulièrement préféré.

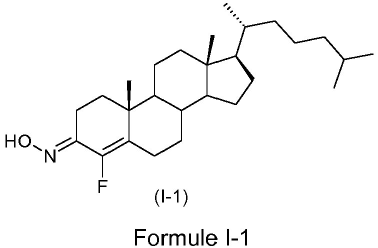

L'introduction d'un tel atome de fluor dans ce composé a permis de façon surprenante de modifier ses propriétés pharmacologiques. Ce composé fluoré (Formule I-1) obtenu sous la forme d'un seul isomère *ANTI*, présente une exposition améliorée lorsqu'il est administré par voie orale.

Selon la présente invention, le 3-oxyimino-6-fluoro-cholest-4-èn (Formule I-2) et le 3-oxyimino-2,2-difluoro-cholest-4-èn (Formule I-3) font partie des composés préférés.

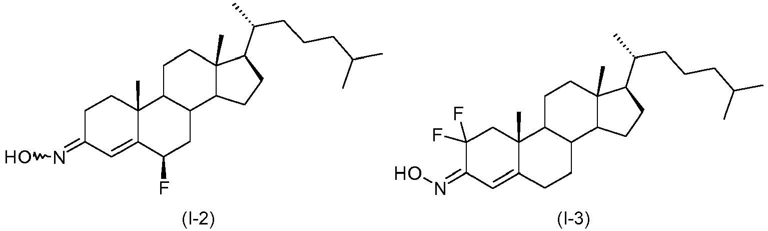

Il est à noter que dans le présent texte, l'atome que peut représenter le terme général "halogène", peut aussi être un isotope, naturel ou synthétique, radioactif comme par exemple pour le fluor, le fluor-18 (¹⁸F). Les composés de formules (I) radiomarqués, particulièrement ceux marqués par l'isotope ¹⁸F, sont très utiles pour l'imagerie médicale, en particulier pour le Positron Emission Tomography (PET) qui est une technique d'imagerie *in vivo* développée pour le diagnostic de maladies, par exemple dans le domaine de l'oncologie, neurologie et cardiologie. Dans le même ordre d'idée on citera pour le brome ou l'iode peut représenter les isotopes radioamarqués brome-75 (⁷⁵Br) et iode-124 (¹²⁴I) respectivement.

Le terme général "halogène" peut également couvrir selon le présent texte les isotopes, naturels ou synthétiques, non radioactifs comme par exemple un isotope non radioactif fluor-19 (¹⁹F), utile pour la recherche biomédicale, et notamment en neurosciences cognitives et en particulier pour la technique d'Imagerie de Résonance Magnétique (IRM).

Les composés objet de la présente invention possèdent de très intéressantes propriétés pharmacologiques. Ils sont doués notamment de remarquables propriétés cytoprotectrices, particulièrement neuroprotectrices, très particulièrement vis à vis des motoneurones, et cardioprotectrices et hépatoprotectrices.

Ces propriétés sont illustrées ci-après dans la partie expérimentale. Elles justifient l'utilisation des composés ci-dessus décrits ainsi que celle de leurs esters et/ou de leurs sels d'addition avec les acides pharmaceutiquement acceptablescomme médicament, particulièrement cytoprotecteurs, particulièrement comme médicaments neuroprotecteurs et/ou cardioprotecteurs et/ou hépatoprotecteurs.

Très particulièrement, les composés selon l'invention présentent une activité remarquable vis à vis des motoneurones, des neurones du système nerveux central, des nerfs moteurs et périphériques.
Ainsi, l'invention a aussi pour objet choisi parmi le 3-oxyimino-4-fluoro-cholest-4-ène ; le 3-oxyimino-6β-fluoro-cholest-4-ène ; le 3-oxyimino-2,2-difluoro-cholest-4-ène ; le 3-oxyimino-2,6-difluoro-cholest-4-ène ; le 3-oxyimino-2α-fluoro-cholest-4-ène ; le 3-oxyimino-25-[méthyl(7-nitro-2,1,3-benzoxadiazol-4-yl)amino]-27-norcholest-4-ène ; le 3-oxyimino-25-((N-(+)-biotinoyl-N-méthyl) amino)-27-norcholest-4-ène ; le 3-oxyimino-19-hydroxy-cholest-4-ène ; le 3-oxyimino-19-biotinyloxy-cholest-4-ène ; le 3-oxyimino-2-méthyl-cholest-4-ène ; le 3-oxyimino-4-méthoxy-cholest-4-ène ; ; ainsi que leurs isomères *SYN*, *ANTI*, lorsqu'ils existent, leurs isomères optiques (énantiomères, diastéréoisomères), lorsqu'ils existent, leurs sels d'addition à un acide ou à une base pharmaceutiquement acceptable, leurs hydrates et leurs solvates, leurs prodrogues, comme médicament. L'invention a donc encore pour objet le 3-oxyimino-4-fluoro-cholest-4-ène ; le 3-oxyimino-6β-fluoro-cholest-4-ène ; le 3-oxyimino-2,2-difluoro-cholest-4-ène ; le 3-oxyimino-2,6-difluoro-cholest-4-ène ; le 3-oxyimino-2α-fluoro-cholest-4-ène ; le 3-oxyimino-25-[méthyl(7-nitro-2,1,3-benzoxadiazol-4-yl)amino]-27-norcholest-4-ène ; le 3-oxyimino-25-((N-(+)-biotinoyl-N-méthyl) amino)-27-norcholest-4-ène ; le 3-oxyimino-19-hydroxy-cholest-4-ène ; le 3-oxyimino-19-biotinyloxy-cholest-4-ène ; le 3-oxyimino-2-méthyl-cholest-4-ène ; le 3-oxyimino-4-méthoxy-cholest-4-ène ; ; ainsi que leurs isomères *SYN, ANTI,* lorsqu'ils existent, leurs isomères optiques (énantiomères, diastéréoisomères), lorsqu'ils existent, leurs sels d'addition à un acide ou à une base pharmaceutiquement acceptable, leurs hydrates et leurs solvates, leurs prodrogues, comme médicament cytoprotecteur.

Les composés selon la présente invention, en raison de leurs propriétés cytoprotectrices, peuvent être utilisés pour la préparation d'un médicament destiné au traitement ou à la prévention de la nécrose et/ou de l'apoptose pathologique et/ou de la nécroptose (médicaments antinécrotiques et/ou antiapoptotiques et/ou antinécroptotique) ou encore au traitement ou à la prévention des affections comme :
▪ les maladies des os, des articulations, du tissu conjonctif et du cartilage, telles que l'ostéoporose, l'ostéomyélite, les arthrites dont par exemple l'ostéoarthrite, l'arthrite rhumatoïde et l'arthrite psoriasique, la nécrose avasculaire, la fibrodysplasie ossifiante progressive, le rachitisme, le syndrome de Cushing ;
▪ les maladies musculaires telles que la dystrophie musculaire, comme par exemple la dystrophie musculaire de Duchenne, les dystrophies myotoniques, les myopathies et les myasthénies ;
▪ les maladies de la peau, telles que les dermatites, l'eczéma, le psoriasis, le vieillissement, ou encore les altérations de la cicatrisation ;
▪ les maladies cardiovasculaires telles que l'ischémie cardiaque et/ou vasculaire, l'infarctus du myocarde, la cardiopathie ischémique, l'insuffisance cardiaque chronique ou aigue, la dysrythmie cardiaque, la fibrillation auriculaire, la fibrillation ventriculaire, la tachycardie paroxystique, l'insuffisance cardiaque, l'anoxie, l'hypoxie, les effets secondaires dus à des thérapies avec des agents anticancéreux ;
▪ les maladies circulatoires telles que l'athérosclérose, les scléroses artérielles, les maladies vasculaires périphériques, les accidents vasculaires cérébraux, les anévrismes ;
▪ les maladies hématologiques et vasculaires telles que : l'anémie, l'amyloïdose vasculaire, les hémorragies, la drépanocytose, le syndrome de la fragmentation des globules rouges, la neutropénie, la leucopénie, l'aplasie médullaire, la pancytopénie, la thrombocytopénie, l'hémophilie ;
▪ les maladies du poumon incluant la pneumonie, l'asthme; les maladies chroniques obstructives des poumons comme par exemple les bronchites chroniques et l'emphysème ;
▪ les maladies du tractus gastro-intestinal, telles que les ulcères ;
▪ les maladies du foie incluant les hépatites virales et les cirrhoses, les maladies du foie dues à des toxines ou des médicaments, les affections susceptibles d'évoluer vers une cirrhose telles que la stéato-hépatite non alcoolique (Non Alcoholic SteatoHepatitis, NASH), la maladie de Wilson, la cholangite sclérosante primitive, ou la cirrhose biliaire primitive ;
▪ les maladies du pancréas comme par exemple les pancréatites aigues ou chroniques ;
▪ les maladies métaboliques telles que les diabètes mellitus et insipide, les thyroïdites ;
▪ les maladies des reins telles que par exemple les désordres rénaux aigus ou la glomérulonéphrite ;
▪ les infections virales et bactériennes telle que la septicémie ;
▪ les intoxications sévères par des agents chimiques, des toxines ou des médicaments ;
▪ les affections dégénératives associées au Syndrome Immuno Déficitaire Acquis (SIDA) ;
▪ les désordres associés au vieillissement, tel que le syndrome du vieillissement accéléré ;
▪ les maladies inflammatoires, telles que la maladie de Crohn, la polyarthrite rhumatoïde ;
▪ les maladies auto-immunes telles que le lupus érythémateux ;
▪ les désordres dentaires tels que ceux aboutissant à la dégradation des tissus comme par exemple les périodontites ;
▪ les maladies ou désordres ophtalmiques incluant les rétinopathies diabétiques, le glaucome, les dégénérescences maculaires, la dégénérescence rétinienne, la rétinite pigmentaire, les trous ou déchirures rétiniennes, le décollement rétinien, l'ischémie rétinienne, les rétinopathies aigues associées à un traumatisme, les dégénérescences inflammatoires, les complications post chirurgicales, les rétinopathies médicamenteuses, la cataracte ;
▪ les désordres des voies auditives, tels que l'otosclérose et la surdité induite par des antibiotiques ;
▪ les maladies associées aux mitochondries (pathologies mitochondriales), telles que l'ataxie de Friedrich, la dystrophie musculaire congénitale avec anomalie mitochondriale structurelle, certaines myopathies (syndrome des MELAS, syndrome de MERFF, syndrome de Pearson), le syndrome de MIDD (diabètes mitochondriaux et surdité), le syndrome de Wolfram, la dystonie.

Très particulièrement, les médicaments selon la présente invention trouvent leur emploi en raison de leurs propriétés neuroprotectrices dans le traitement ou à la prévention des affections neurodégénératives, comme par exemple la maladie de Huntington, les maladies chroniques neurodégénératives, avantageusement les maladies neurodégénératives chroniques démyélinisantes, héréditaires ou sporadiques, les lésions neuronales liées au vieillissement, les neuropathies périphériques, héréditaires ou lésionnelles, les neuropathies diabétiques ou induites par les traitements anticancéreux, les traumatismes du cerveau, des nerfs périphériques ou de la moelle épinière, les ischémies du cerveau ou de la moelle épinière, les épilepsies, les dégénérescences héréditaires, lésionnelles ou liées au vieillissement des neurones sensoriels de la vision ou les dégénérescences du nerf optique, les dégénérescences héréditaires, traumatiques ou liées au vieillissement des neurones sensoriels de l'ouïe, les atrophies lobaires et les démences vasculaires, et notamment les amyotrophies spinales, la sclérose latérale amyotrophique et les pathologies dues aux traumatismes de la moelle épinière ou des nerfs moteurs périphériques.

Ils trouvent notamment en raison de leurs propriétés neuroprotectrices vis à vis des motoneurones, leur emploi particulièrement dans le traitement des amyotrophies spinales, notamment de la sclérose latérale amyotrophique ou des amyotrophies spinales infantiles, et dans le traitement des traumatismes de la moelle épinière ou des nerfs moteurs périphériques comme évoqué ci-dessus.

En général la dose journalière du composé sera la dose minimum pour obtenir l'effet thérapeutique. Cette dose dépendra des différents facteurs cités auparavant. Les doses des composés, ci-dessus décrits y compris le 3-oxyimino-4-chloro-cholest-4-ène, 3-oxyimino-4-bromo-cholest-4-ène, le 3-oxyimino-6-éthoxy-cholest-4,6-diène et 3-oxyimino-4-méthyl-cholest-4-ène pourront être en général comprises entre 0,001 à 100 mg par kilo par jour pour l'homme.

Si nécessaire, la dose journalière peut être administrée en deux, trois, quatre, cinq, six ou plus, prises par jour ou par sous-doses multiples administrées par intervalles appropriés pendant la journée.

La quantité choisie dépendra de multiples facteurs, en particulier de la voie d'administration, de la durée d'administration, du moment de l'administration, de la vitesse d'élimination du composé, du ou des différents produits utilisés en combinaison avec le composé, de l'âge, du poids et de la condition physique du patient, ainsi que de son histoire médicale, et de toutes autres informations connues en médecine.

La prescription du médecin traitant pourra commencer à des doses inférieures à celles généralement utilisées, puis ces doses seront progressivement augmentées afin de mieux maîtriser l'apparition d'éventuels effets secondaires.
L'invention a aussi pour objet les compositions pharmaceutiques qui comprennent au moins un composé choisi parmi le 3-oxyimino-4-fluoro-cholest-4-ène ; le 3-oxyimino-6β-fluoro-cholest-4-ène ; le 3-oxyimino-2,2-difluoro-cholest-4-ène ; le 3-oxyimino-2,6-difluoro-cholest-4-ène ; le 3-oxyimino-2α-fluoro-cholest-4-ène ; le 3-oxyimino-25-[méthyl(7-nitro-2,1,3-benzoxadiazol-4-yl)amino]-27-norcholest-4-ène ; le 3-oxyimino-25-((N-(+)-biotinoyl-N-méthyl) amino)-27-norcholest-4-ène ; le 3-oxyimino-19-hydroxy-cholest-4-ène ; le 3-oxyimino-19-biotinyloxy-cholest-4-ène ; le 3-oxyimino-2-méthyl-cholest-4-ène ; le 3-oxyimino-4-méthoxy-cholest-4-ène ; ; ainsi que leurs isomères *SYN, ANTI,* lorsqu'ils existent, leurs isomères optiques (énantiomères, diastéréoisomères), lorsqu'ils existent, leurs sels d'addition à un acide ou à une base pharmaceutiquement acceptable, leurs hydrates et leurs solvates, leurs prodrogues, à titre de principe actif.

Dans ces compositions, le principe actif est avantageusement présent à des doses physiologiquement efficaces; les compositions précitées peuvent comprendre notamment une dose neuroprotectrice efficace d'au moins un principe actif ci-dessus.

A titre de médicaments, lesdits composés que leurs esters et/ou leurs sels d'addition avec les acides pharmaceutiquement acceptables peuvent être incorporés dans des compositions pharmaceutiques destinées à la voie digestive ou parentérale.

Les compositions pharmaceutiques selon l'invention peuvent comprendre en outre au moins un autre ingrédient thérapeutiquement actif, pour une utilisation simultanée, séparée ou étalée dans le temps, notamment lors d'un traitement chez un sujet atteint d'une pathologie ou d'un traumatisme lié à la dégénérescence ou à la mort des cellules particulièrement des cellules cardiaques et/ou des motoneurones tel que défini ci-dessus.

Les compositions pharmaceutiques ou médicaments selon l'invention comprennent avantageusement un ou plusieurs excipients ou véhicules inertes, c'est à dire pharmaceutiquement inactifs et non toxiques. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, les cyclodextrines, le polysorbate 80, le mannitol, la gélatine, le lactose, des huiles végétales ou animales, l'acacia, etc. Les compositions peuvent être formulées sous forme de suspension injectable, de gels, huiles, comprimés, suppositoires, poudres, gélules, capsules, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

L'administration peut être réalisée par toute méthode connue de l'homme du métier, de préférence par voie orale ou par injection, typiquement par voie intra-péritonéale, intra-cérébrale, intra-thécale, intra-veineuse, intra-artérielle ou intramusculaire. L'administration par voie orale est préférée. S'agissant d'un traitement à long terme, la voie d'administration préférée sera sublinguale, orale ou transcutanée.

Pour les injections, les composés sont généralement conditionnés sous forme de suspensions liquides, qui peuvent être injectées au moyen de seringues ou de perfusions, par exemple. Il est entendu que le débit et/ou la dose injectée, ou de manière générale la dose à administrer, peuvent être adaptés par l'homme du métier en fonction du patient, de la pathologie, du mode d'administration, etc. Il est entendu que des administrations répétées peuvent être réalisées, éventuellement en combinaison avec d'autres ingrédients actifs ou tout véhicule acceptable sur le plan pharmaceutique (tampons, solutions saline, isotonique, en présence d'agents stabilisants, etc.).

L'invention est utilisable chez les mammifères, notamment chez l'être humain.

La présente invention a encore pour objet un procédé de préparation d'une composition ci-dessus décrite, caractérisé en ce que l'on mélange, selon des méthodes connues en elles mêmes, le ou les principes actifs avec des excipients acceptables, notamment pharmaceutiquement acceptables. L'utilisation d'un composé choisi parmi le 3-oxyimino-4-fluoro-cholest-4-ène ; le 3-oxyimino-6β-fluoro-cholest-4-ène ; le 3-oxyimino-2,2-difluoro-cholest-4-ène ; le 3-oxyimino-2,6-difluoro-cholest-4-ène ; le 3-oxyimino-2α-fluoro-cholest-4-ène ; le 3-oxyimino-25-[méthyl(7-nitro-2,1,3-benzoxadiazol-4-yl)amino]-27-norcholest-4-ène ; le 3-oxyimino-25-((N-(+)-biotinoyl-N-méthyl) amino)-27-norcholest-4-ène ; le 3-oxyimino-19-hydroxy-cholest-4-ène ; le 3-oxyimino-19-biotinyloxy-cholest-4-ène ; le 3-oxyimino-2-méthyl-cholest-4-ène ; le 3-oxyimino-4-méthoxy-cholest-4-ène ; ainsi que leurs isomères *SYN, ANTI,* lorsqu'ils existent, leurs isomères optiques (énantiomères, diastéréoisomères), lorsqu'ils existent, leurs sels d'addition à un acide ou à une base pharmaceutiquement acceptable, leurs hydrates et leurs solvates, leurs prodrogues comme médicament destiné au traitement ou à la prévention des pathologies ou traumatismes liés à la dégénérescence ou à la mort des cellules, particulièrement des cellules cardiaques et/ou des neurones, que celles-ci soient naturelles ou accidentelles est décrite. L'utilisation d'un composé choisi parmi le 3-oxyimino-4-fluoro-cholest-4-ène ; le 3-oxyimino-6β-fluoro-cholest-4-ène ; le 3-oxyimino-2,2-difluoro-cholest-4-ène ; le 3-oxyimino-2,6-difluoro-cholest-4-ène ; le 3-oxyimino-2α-fluoro-cholest-4-ène ; le 3-oxyimino-25-[méthyl(7-nitro-2,1,3-benzoxadiazol-4-yl)amino]-27-norcholest-4-ène ; le 3-oxyimino-25-((N-(+)-biotinoyl-N-méthyl) amino)-27-norcholest-4-ène ; le 3-oxyimino-19-hydroxy-cholest-4-ène ; le 3-oxyimino-19-biotinyloxy-cholest-4-ène; le 3-oxyimino-2-méthyl-cholest-4-ène ; le 3-oxyimino-4-méthoxy-cholest-4-ène; ainsi que leurs isomères *SYN, ANTI,* lorsqu'ils existent, leurs isomères optiques (énantiomères, diastéréoisomères), lorsqu'ils existent, leurs sels d'addition à un acide ou à une base pharmaceutiquement acceptable, leurs hydrates et leurs solvates, leurs prodrogues comme médicament destiné au traitement ou à la prévention de la nécrose et/ou de l'apoptose pathologique et/ou de la nécroptose (médicaments antinécrotiques et/ou antiapoptotiques et/ou antinécroptotiques) ou encore au traitement ou à la prévention des affections comme :
▪ les maladies des os, des articulations, du tissu conjonctif et du cartilage, telles que l'ostéoporose, l'ostéomyélite, les arthrites dont par exemple l'ostéoarthrite, l'arthrite rhumatoïde et l'arthrite psoriasique, la nécrose avasculaire, la fibrodysplasie ossifiante progressive, le rachitisme, le syndrome de Cushing ;
▪ les maladies musculaires telles que la dystrophie musculaire, comme par exemple la dystrophie musculaire de Duchenne, les dystrophies myotoniques, les myopathies et les myasthénies ;
▪ les maladies de la peau, telles que les dermatites, l'eczéma, le psoriasis, le vieillissement, ou encore les altérations de la cicatrisation;
▪ les maladies cardiovasculaires telles que l'ischémie cardiaque et/ou vasculaire, l'infarctus du myocarde, la cardiopathie ischémique, l'insuffisance cardiaque chronique ou aigue, la dysrythmie cardiaque, la fibrillation auriculaire, la fibrillation ventriculaire, la tachycardie paroxystique, l'insuffisance cardiaque, l'anoxie, l'hypoxie, les effets secondaires dus à des thérapies avec des agents anticancéreux ;
▪ les maladies circulatoires telles que l'athérosclérose, les scléroses artérielles, et les maladies vasculaires périphériques, les accidents vasculaires cérébraux, les anévrismes ;
▪ les maladies hématologiques et vasculaires telles que : l'anémie, l'amyloïdose vasculaire, les hémorragies, la drépanocytose, le syndrome de la fragmentation des globules rouges, la neutropénie, la leucopénie, l'aplasie médullaire, la pancytopénie, la thrombocytopénie, l'hémophilie ;
▪ les maladies du poumon incluant la pneumonie, l'asthme ; les maladies chroniques obstructives des poumons comme par exemple les bronchites chroniques et l'emphysème ;
▪ les maladies du tractus gastro-intestinal, telles que les ulcères ;
▪ les maladies du foie incluant les hépatites virales et les cirrhoses, les maladies du foie dues à des toxines ou des médicaments, les affections susceptibles d'évoluer vers une cirrhose telles que la stéato-hépatite non alcoolique (en anglais non alcoholic steatohepatitis, NASH), la maladie de Wilson, la cholangite sclérosante primitive, ou la cirrhose biliaire primitive
▪ les maladies du pancréas comme par exemple les pancréatites aigues ou chroniques ;
▪ les maladies métaboliques telles que les diabètes mellitus et insipide, les thyroïdites ;
▪ les maladies des reins telles que par exemple les désordres rénaux aigus ou la glomérulonéphrite ;
▪ les infections virales et bactériennes telle que la septicémie ;
▪ les intoxications sévères par des agents chimiques, des toxines ou des médicaments ;
▪ les affections dégénératives associées au Syndrome Immuno Déficitaire Acquis (SIDA) ;
▪ les désordres associés au vieillissement, tel que le syndrome du vieillissement accéléré ;
▪ les maladies inflammatoires, telles que la maladie de Crohn, la polyarthrite rhumatoïde ;
▪ les maladies auto-immunes telles que le lupus érythémateux ;
▪ les désordres dentaires tels que ceux aboutissant à la dégradation des tissus comme par exemple les périodontites ;
▪ les maladies ou désordres ophtalmiques incluant les rétinopathies diabétiques, le glaucome, les dégénérescences maculaires, la dégénérescence rétinienne, la rétinite pigmentaire, les trous ou déchirures rétiniennes, le décollement rétinien, l'ischémie rétinienne, les rétinopathies aigues associées à un traumatisme, les dégénérescences inflammatoires, les complications post chirurgicales, les rétinopathies médicamenteuses, la cataracte ;
▪ les désordres des voies auditives, tels que l'otosclérose et la surdité induite par des antibiotiques ;
▪ les maladies associées aux mitochondries (pathologies mitochondriales), telles que l'ataxie de Friedrich, la dystrophie musculaire congénitale avec anomalie mitochondriale structurelle, certaines myopathies (syndrome des MELAS, syndrome de MERFF, syndrome de Pearson), le syndrome de MIDD (diabètes mitochondriaux et surdité), le syndrome de Wolfram, la dystonie, et particulièrement
▪ des maladies neurodégénératives comme par exemple la maladie de Huntington, les maladies chroniques neurodégénératives, avantageusement les maladies chroniques démyélinisantes et neurodégénératives,héréditaires ou sporadiques, notamment la sclérose en plaque et les leucodystrophies, les lésions neuronales liées au vieillissement, les neuropathies périphériques héréditaires ou lésionnelles, la maladie de Charcot-Marie-Tooth, les neuropathies diabétiques ou induites par les traitements anticancéreux, les épilepsies, les traumatismes du cerveau, des nerfs périphériques ou de la moelle épinière, les ischémies du cerveau ou de la moelle épinière, les dégénérescences héréditaires, lésionnelles ou liées au vieillissement des neurones sensoriels de la vision ou les dégénérescences du nerf optique, les dégénérescences héréditaires, traumatiques ou liées au vieillissement des neurones sensoriels de l'ouïe, les atrophies lobaires et les démences vasculaires, les maladies et traumatismes liés à la dégénérescence des motoneurones et plus particulièrement les amyotrophies spinales particulièrement infantiles, la sclérose latérale amyotrophique, la sclérose en plaques et les traumatismes de la moelle épinière ou des nerfs moteurs périphériques est décrite. L'utilisation d'un composé choisi parmi le 3-oxyimino-4-fluoro-cholest-4-ène ; le 3-oxyimino-6β-fluoro-cholest-4-ène ; le 3-oxyimino-2,2-difluoro-cholest-4-ène ; le 3-oxyimino-2,6-difluoro-cholest-4-ène ; le 3-oxyimino-2α-fluoro-cholest-4-ène ; le 3-oxyimino-25-[méthyl(7-nitro-2,1,3-benzoxadiazol-4-yl)amino]-27-norcholest-4-ène ; le 3-oxyimino-25-((N-(+)-biotinoyl-N-méthyl) amino)-27-norcholest-4-ène ; le 3-oxyimino-19-hydroxy-cholest-4-ène ; le 3-oxyimino-19-biotinyloxy-cholest-4-ène ; le 3-oxyimino-2-méthyl-cholest-4-ène ; le 3-oxyimino-4-méthoxy-cholest-4-ène ; ainsi que leurs isomères *SYN, ANTI,* lorsqu'ils existent, leurs isomères optiques (énantiomères, diastéréoisomères), lorsqu'ils existent, leurs sels d'addition à un acide ou à une base pharmaceutiquement acceptable, leurs hydrates et leurs solvates, leurs prodrogues comme médicament destiné au traitement des amyotrophies spinales, particulièrement infantiles, et des scléroses latérales amyotrophiques est décrite.
La mise en oeuvre de ces médicaments comprend habituellement l'administration aux patients, particulièrement aux mammifères, tout particulièrement aux êtres humains, d'une quantité thérapeutiquement efficace d'un composé choisi parmi le 3-oxyimino-4-fluoro-cholest-4-ène ; le 3-oxyimino-6β-fluoro-cholest-4-ène ; le 3-oxyimino-2,2-difluoro-cholest-4-ène ; le 3-oxyimino-2,6-difluoro-cholest-4-ène ; le 3-oxyimino-2α-fluoro-cholest-4-ène ; le 3-oxyimino-25-[méthyl(7-nitro-2,1,3-benzoxadiazol-4-yl)amino]-27-norcholest-4-ène ; le 3-oxyimino-25-((N-(+)-biotinoyl-N-méthyl) amino)-27-norcholest-4-ène ; le 3-oxyimino-19-hydroxy-cholest-4-ène ; le 3-oxyimino-19-biotinyloxy-cholest-4-ène ; le 3-oxyimino-2-méthyl-cholest-4-ène ; le 3-oxyimino-4-méthoxy-cholest-4-ène ; ainsi que leurs isomères *SYN, ANTI,* lorsqu'ils existent, leurs isomères optiques (énantiomères, diastéréoisomères), lorsqu'ils existent, leurs sels d'addition à un acide ou à une base pharmaceutiquement acceptable, leurs hydrates et leurs solvates, leurs prodrogues comme, en particulier pour augmenter la survie des cellules, particulièrement des cellules cardiaques et/ou des neurones ou favoriser la croissance axonale. Une méthode de traitement des maladies précitées, notamment neurodégénératives, et notamment une méthode de traitement de pathologies ou traumatismes liés à la dégénérescence ou à la mort des neurones, chez des mammifères (en général des patients) atteints de telles pathologies ou traumatismes, comprenant l'administration à ces mammifères d'une quantité thérapeutiquement efficace d'un composé de formule I, y compris le 3-oxyimino-4-chloro-cholest-4-ène, le 3-oxyimino-4-bromo-cholest-4-ène, le 3-oxyimino-6-éthoxy-cholest-4,6-diène et le 3-oxyimino-4-méthyl-cholest-4-ène en particulier pour augmenter la survie des neurones ou favoriser la croissance axonale est décrite. Une méthode de traitement d'une des affections ci-dessus décrites et notamment des pathologies ou traumatismes liés à la dégénérescence ou à la mort des motoneurones, chez des mammifères (en général des patients) atteints de tels pathologies ou traumatismes, comprenant l'administration à ces mammifères d'une quantité thérapeutiquement efficace d'un composé de formule I, y compris le 3-oxyimino-4-chloro-cholest-4-ène, le 3-oxyimino-4-bromo-cholest-4-ène, le 3-oxyimino-6-éthoxy-cholest-4,6-diène et le 3-oxyimino-4-méthyl-cholest-4-ène en particulier pour augmenter la survie des neurones est décrite. Plus spécifiquement, les pathologies liées à la dégénérescence ou à la mort des motoneurones sont la sclérose latérale amyotrophique ou les amyotrophies spinales infantiles. Selon une autre variante, l'invention concerne aussi des composés comportant un groupement de marquage détectable et/ou visualisable directement ou indirectement par les techniques de détection et/ou de visualisation connues de l'homme de l'art telles que la technique de la microscopie de fluorescence ou la technique tirant profit de la très forte affinité des avidines (streptavidine ou neutravidine) pour la biotine (Ka ∼ 10⁷M).

Au sens général du terme, on entend par "marquage" une entité telle qu'un isotope radioactif ou une entité non isotopique telle qu'un agent fluorophore, un colorant, un haptène, la biotine, etc.

Le terme fluorophore se réfère en général à la particularité qu'à une substance d'être fluorescente, c'est-à-dire d'absorber de l'énergie lumineuse (lumière d'excitation) lorsqu'elle est excitée par une source énergétique et de la restituer rapidement sous forme de lumière fluorescente (lumière d'émission). Cette particularité d'être fluorophore permet d'envisager l'utilisation de telle substance comme marqueur fluorescent dans des systèmes biologiques (membranes, cellules, neurones, mitochondries, etc) pour réaliser par exemple l'imagerie des cellules étudiées.

La biotine est une coenzyme, aussi appelée Vitamine H, synthétisée par les plantes, les bactéries et certains champignons. La biotine est détectée au moyen d'avidines (streptavidine ou neutravidine) qui possèdent une très forte affinité pour la biotine (Ka ∼ 10⁷M). La structure de la biotine est la suivante :

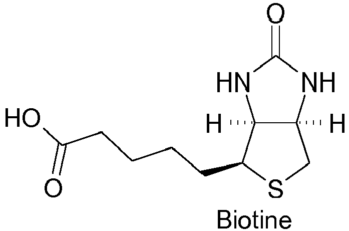

Il est connu dans l'art antérieur que l'incorporation d'un groupement biotine à une substance biologiquement active permet d'isoler et/ou identifier les cibles protéiques ou autres composés non protéiniques susceptibles d'interagir avec ladite substance active en utilisant l'une des approches tirant profit de la très forte affinité des avidines pour la biotine. Les cibles protéiques ou autres composés non protéiniques ainsi isolés sont par exemple caractérisés par la spectrométrie de masse. Parmi les applications connues de l'homme de l'art, on peut citer par exemple: les tests diagnostics utilisant des composés biotinylés ; le test ELISA (Enzyme-Linked Immunosorbent Assay) qui emploie des antibiotiques biotinylés ; la chromatographie d'affinité basée sur l'utilisation d'une colonne d'avidine immobilisée sur laquelle on charge des composés biotinylés ; les techniques d'analyse protéomique (électrophorèse 2D et spectrométrie de masse), etc.

Le groupement de marquage selon la présente invention peut être choisi parmi la biotine ou un groupement fluorophore tel que le 7-nitrobenz-2-oxa-1,3-diazol-4-yl (formule D); BODIPY® fluorophore ; anthracène et fluorescéine. De préférence, le groupement de marquage selon la présente invention est choisi parmi la biotine et le groupement fluorophore 7- nitrobenz-2-oxa-1,3-diazol-4-yl (formule D). 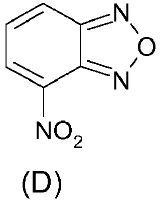 Les composés selon la présente invention marqués par la biotine ou par un groupement fluorophore sont des sondes très utiles pour :
- visualiser et/ou détecter les cellules qui sont en contact avec les composés marqués,
- étudier leur distribution dans un organisme vivant, humain ou animal, et leur localisation dans les compartiments cellulaires (membranes, cellules, neurones, mitochondrie, noyau, réticulum endoplasmique, golgi, lysosomes, endosomes et autres organelles, etc),
- mettre en oeuvre une méthode de détection de protéines ou autres composés non protéiniques susceptibles d'interagir avec lesdits composés marqués
- identifier leur(s) cible(s) moléculaire(s),
- étudier les interactions molécules-protéine d'un point de vue moléculaire,
- détecter les anticorps monoclonaux spécifiques de l'oxime de la cholest-4-èn-3-one ou des ses dérivés,
- développer et réaliser des essais de binding permettant, entre autre, l'optimisation de ligands plus affinés pour la cible en question,
- développer des méthodes de dosage,
- développer de nouveaux outils de criblage de ligands
L'incorporation d'un tel groupement de marquage n'a pas provoqué de perte de l'activité biologique des composés marqués selon la présente invention et a permis l'utilisation de ces composés marqués en tant que sondes en particulier en tant que traceurs ou agents de marquage.

Par conséquent, un autre objet selon l'invention est de fournir des composés de formule (I) comportant un groupement de marquage choisi parmi le groupe NBD et la biotine. L'utilisation en tant que sondes en particulier en tant que traceurs ou agents de marquage, des composés de formule I, pour lesquels :
- R₆ peut représenter un groupement répondant à la formule (A) suivante :

   -CH₂-Q-R^{c} (A)

   dans laquelle
   - Q peut représenter un atome d'oxygène ou un groupe NR^{a}, R^{a} est tel que décrit précédemment, et
   - R^{c} peut représenter un groupement répondant à la formule (C) ou (D) 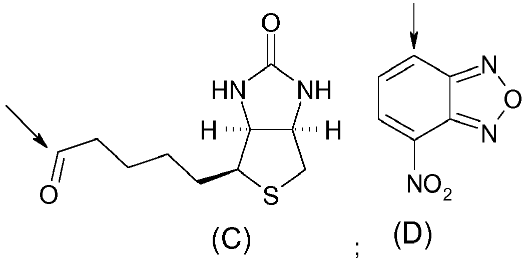 ou,
- R₁₃ peut représenter un groupement répondant à la formule (E) suivante :

   R₁₄-X-R₁₅ (E)

   dans laquelle :
   - R₁₄ peut représenter le groupement G₇ suivant 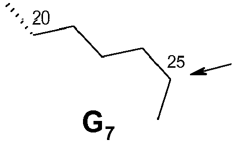
   - X peut représenter un atome d'oxygène, ou NR^{a}, R^{a} est tel décrit précédemment, en particulier, NH ou NCH₃, et
   - R₁₅ peut représenter un groupement répondant à la formule (C) ou (D) suivante est décrite

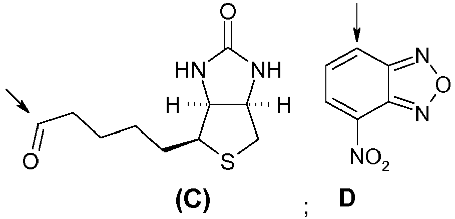

Les composés particulièrement préférés comme traceurs ou agents de marquage selon l'invention sont représentés par les formules (III), (IV), (V) suivantes : 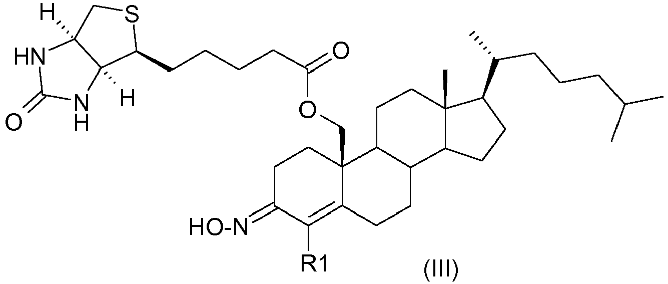 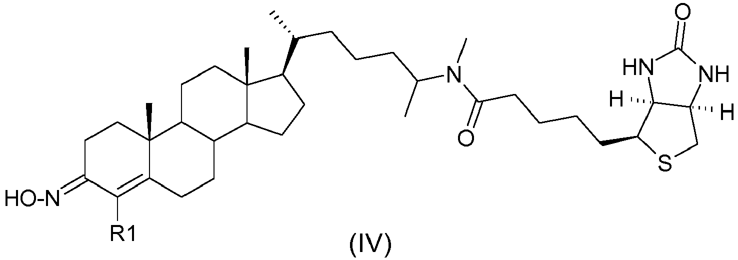 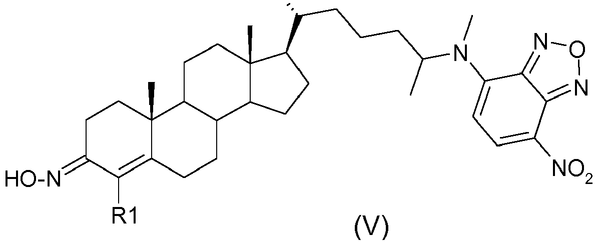 formules (III), (IV) ou (V) dans lesquelles le substituant R₁ peut être tel que décrit précédemment. Préférentiellement, le substituant R₁ est un atome d'hydrogène.

Les composés de formule (I) selon l'invention peuvent être obtenus par différents procédés de synthèse faisant appel notamment à la réaction d'oximation des cétones qui est bien connue de l'homme du métier. Le schéma ci-dessous est illustratif du procédé utilisé pour la préparation des composés de formule (I)

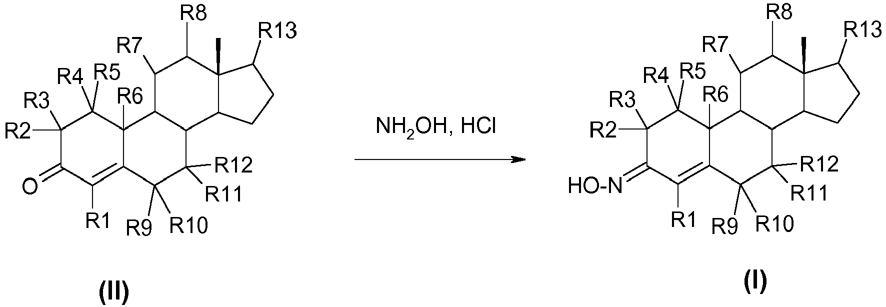

A titre d'exemple, un procédé particulièrement approprié pour l'obtention des composés de formule (I) consiste à faire réagir :
(i) un composé de formule (II) dans laquelle les groupements R₁ à R₁₃ sont tels que définis précédemment, avec
(ii) un halogénure d'hydroxylamine comme le chlorhydrate d'hydroxylamine.

Ce procédé peut être avantageusement réalisé dans un solvant adapté comme la pyridine.

Les composés de formule (I) peuvent être isolés du milieu réactionnel par différentes méthodes bien connues de l'homme du métier. Optionnellement, les composés de formule (I) peuvent être transformés en l'un de leurs sels pharmaceutiquement acceptables. Les composés de formule (II) utilisés comme produits de départ pour l'obtention des composés de formules (I) sont disponibles commercialement ou sont préparés par des méthodes connues de l'homme du métier.

Les exemples suivants illustrent la présente invention mais sans la limiter. Les structures des composés décrits dans les exemples et dans les préparations ont été déterminées selon les techniques usuelles (résonance magnétique nucléaire, spectroscopie de masse, etc).

### Abréviations :

EP : éther de pétrole
AE : acétate d'éthyle
s : singulet
d : doublet
t : triplet
sept : septuplet

### Exemple 1 - Préparation de composés de formule (II)

### Exemple 1a - Synthèse de la 4-fluoro-cholest-4-èn-3-one

La 4-fluoro-cholest-4-èn-3-one a été préparée selon la méthode décrite dans les articles suivants : Toyota, A. & al. Chemical & Pharmaceutical Bulletin (1994), 42(3), 459-61 ; et Nakanishi, S. & al : Chemistry & Industry (London, United Kingdom) (1960), 1136-7.

### Exemple 1b - Synthèse de la 6β-fluoro-cholest-4-èn-3-one

La 6β-fluoro-cholest-4-èn-3-one a été préparée selon l'une des méthodes décrites dans les articles suivants : Thomas, M. G. et al. Journal of the Chemical Society, Perkin Transactions 1: Organic and Bio-Organic Chemistry (1999), (21), 3191-3198; Poss, A. J. et al. Tetrahedron Letters (1995), 36(27), 4721-4; Edmunds, J.J. et al. Journal of the Chemical Society, Chemical Communications (1989), (13), 881-3. Salmond, W. G. et al. Ger. Offen. (1983), DE 3225747; Nakanishi, S. et al. Journal of the American Chemical Society (1959), 81 5259-60.

### Exemple 1c - Synthèse de la 2,2-difluoro-cholest-4-èn-3-one et de la 2,6-difluoro-cholest-4-èn-3-one

Dans un ballon sont ajoutés 10 g (26 mmol) de cholest-4-èn-3-one en suspension dans 260 mL de méthanol puis 17.4 g (27,2 mmol) de 1-fluoro-4-hydroxy-1,4-diazoniabicyclo[2,2,2]octane bis(tétrafluoroborate) à 50% en masse sur alumine. Le milieu est agité au reflux pendant 16h et 7,7g du réactif de fluoration sont rajoutés ; l'agitation est poursuivie 5h au reflux et 4 jours à température ambiante. Le milieu réactionnel est alors concentré sous vide et le résidu est repris dans du dichlorométhane. Après filtration, la solution obtenu est concentrée sous vide et le résidu est repris dans 500 mL d'acide chlorhydrique à 10% dans de l'acétonitrile. La suspension est agitée 2h à température ambiante et filtrée. Le précipité est lavé à l'acétate d'éthyle et le filtrat est extrait à l'acétate d'éthyle et la phase organique est concentrée sous vide.

Une purification du précipité par flash chromatographie sur gel de silice (éluant EP/AE, gradient de 100% EP à 80/20 EP/AE) puis par HPLC semi-préparative permet d'isoler 17,5 mg du 2,6-difluoro-cholest-4-èn-3-one et 23,1 mg de 2,2-difluoro-4-cholestèn-3-one.
- Le résidu obtenu à partir du filtrat est purifié par flash chromatographie sur gel de silice (éluant EP/AE, gradient de 100% EP à 80/20 EP/AE) puis par HPLC semi-préparative permet d'isoler 82 mg supplémentaire de 2,2-difluoro-cholest-4-èn-3-one.
- 2,6-difluoro-cholest-4-èn-3-one
   RMN-¹H (CDCl₃) : δ (ppm) 6,11 (d, 1H, 4-CH), 5,21 (dddd, 1H, 2-CH), 4,86 (ddd, 1H, 6-CH), 0,71 (s, 3H, 18-CH₃)..
   LC/UV/MS (254 nm) : T_{R} = 5,82 min, m/z = 421 [M+H]⁺
- 2,2-difluoro-cholest-4-èn-3-one
   RMN-¹H (CDCl₃):□ δ (ppm) 6,26 (d, 1H, 4-CH), 0,73 (s, 3H, 18-CH₃)
   RMN-¹⁹F (CDCl₃) : δ □(ppm, non calibré) -86,33 (ddd, 2-CFₐ), -101,04 (d, 2-CF_{b})
   LC/UV/MS (254 nm) : T_{R} = 6,48 min, m/z = 421 [M+H]⁺

### Exemple 1d - Synthèse de la 2α-fluoro-cholest-4-èn-3-one

Dans un ballon sont ajoutés 5 g (13 mmol) de cholest-4-èn-3-one en suspension dans 130 mL de méthanol puis 8,7 g (13,6 mmol) 1-fluoro-4-hydroxy-1,4-diazoniabicyclo[2,2,2]octane bis(tétrafluoroborate) à 50% en masse sur alumine. Le milieu est agité au reflux pendant 3h et 4,3g du réactif de fluoration sont ajoutés ; l'agitation est poursuivie 3h au reflux et 1 nuit à température ambiante. Le milieu réactionnel est alors concentré sous vide et le résidu est repris dans du dichlorométhane. Après filtration, la solution obtenu est concentrée sous vide et le résidu est repris dans 250 mL d'acide chlorhydrique à 10% dans de l'acétonitrile. La suspension est agitée 1h à température ambiante et filtrée. Le précipité est lavé à l'acétate d'éthyle et le filtrat est extrait à l'acétate d'éthyle et la phase organique est concentrée sous vide.

Le résidu obtenu est purifié par flash chromatographie sur gel de silice (éluant EP/AE, gradient de 100% EP à 95/5 EP/AE) puis par HPLC semi-préparative permet d'isoler 30 mg de 2α-fluoro-cholest-4-èn-3-one.
LC/UV/MS (254 nm) : T_{R} = 6,27 min (syn/anti), m/z = 403 [M+H]⁺

### Exemple 1e - Synthèse de la 25-fluoro-cholest-4-èn-3-one

Dans un ballon à 0°C sont introduits 100 mg (247 mmol) de 25-fluoro-5-cholestèn-3β-ol et 15 mL d'acétone puis 160 µL de réactif de Jones sont ajoutés. Le milieu est agité 9 minutes à 0°C puis la réaction est stoppée par ajout d'éthanol. Après concentration sous vide à une température inférieure à 30°C, le résidu est repris dans 10 mL d'éthanol et 100 µL d'une solution 1N d'acide chlorhydrique sont ajoutés. Le milieu est agité 15 minutes à 50-60°C puis concentré sous vide. Le résidu obtenu est repris dans l'eau et extrait à l'acétate d'éthyle ; la phase organique est séparée, lavée à l'eau, séchée sur sulfate de magnésium anhydre et concentrée sous vide. Après purification par flash chromatographie sur gel de silice (éther de pétrole puis éther de pétrole / acétate d'éthyle 95/5), 69 mg de l'énone sont obtenus avec un rendement de 69%.
LC/UV/MS (254 nm) : T_{R} = 5,41 min, m/z = 403 [M+H]⁺

### Exemple 1f - Synthèse de la 25-[méthyl(7-nitro-2,1,3-benzoxadiazol-4-yl) amino]-27-norcholest-5-èn-3-one , et de la 25-((N-(+)-biotinoyl-N-méthyl)amino)-27-norcholest-4-èn-3-one

### Etape 1 : Synthèse du tert-butyldiméthylsilyl-25-(N-méthylamino)-27-norcholestérol

Dans un ballon, sont introduits 2,83 g (5,65 mmol) de tert-butyldiméthylsilyl-25-céto-27-norcholestérol⁽¹⁾, 3,62 g (53,67 mmol) de chlorhydrate de N-méthylamine et 355 mg (5,65 mmol) de cyanoborohydrure de sodium dans 40 mL de méthanol. La solution est agitée une nuit à température ambiante puis le milieu est concentré sous vide. Le résidu est repris dans une solution 2M de carbonate de sodium dans l'eau et extrait 3 fois au dichlorométhane. Les phases organiques sont réunies, lavées par une solution saturée de chlorure de sodium, séchées sur sulfate de magnésium anhydre, filtrées et concentrées sous vide. Le produit obtenu est utilisé tel quel sans purification.
LC/DEDL/MS : T_{R} = 10,42 min, *m*/*z* = 516 [M+H]⁺
RMN-¹H (CDCl₃) : δ (ppm) 5,29-5,33 (massif, 1H, 6-CH), 3,47 (m, 1H, 3-CH), 2,10-2,55 (massif, 4H, 25-CH et NCH₃), 0,67 (s, 3H, 18-CH₃), 0,05 (s, 6H, Si(CH₃)₂)
(1) Ferraboschi, P. et al., Tetrahedron Asymmetry (1999), 10(13), 2497-2500; Ferraboschi, P. et al., Tetrahedron: Asymmetry (1998), 9(13), 2193-2196. Okamoto, M. et al. *Jpn. Kokai Tokkyo Koho* (1997), 15 pp. CODEN: JKXXAF JP 09249691 A 19970922; Satsangi, R. K. et al. Analyst (Cambridge, United Kingdom) (1992), 117(6), 953-7.

### Etape 2 : Synthèse du 25-(N-méthylamino)-27-norcholestérol

Dans un ballon, sont introduits 2 g (3,87 mmol) de tert-butyldiméthylsilyl-25-(N-méthylamino)-27-norcholestérol obtenu à l'étape 1 dans 12 mL de dichlorométhane. 4 mL d'une solution 4N de chlorure d'hydrogène dans du dioxane sont ajoutés goutte-à-goutte. Le milieu réactionnel est agité 1 heure à température ambiante puis filtré sur fritté. Le filtrat est basifié par ajout d'une solution de soude et extrait 3 fois au dichlorométhane. Les phases organiques sont réunies, séchées sur sulfate de magnésium anhydre, filtrées et concentrées sous vide. Le produit obtenu est utilisé tel quel sans purification.
LC/DEDL/MS : T_{R} = 1,85 et 2,08 min, *m*/*z* = 402 [M+H]⁺
RMN-¹H (CDCl₃) : δ (ppm) 5,29-5,40 (massif, 1H, 6-CH), 3,50 (m, 1H, 3-CH), 2,10-2,65 (massif, 4H, 25-CH et NCH₃), 0,67 (s, 3H, 18-CH₃).

### Etape 3 : Synthèse du 25-(N-méthylamino)-27-norcholest-4-èn-3-one

Dans un ballon à 0°C sont introduits 870 mg (2,16 mmol) de 25-(N-méthylamino)-27-norcholestérol obtenus à l'étape 1, 15 mL d'acétone et 6 mL de dichlorométhane puis 1,1 mL de réactif de Jones sont ajoutés. Le milieu est agité 20 minutes à 0°C puis la réaction est stoppée par ajout de 2 mL d'éthanol. Après concentration sous vide à une température inférieure à 30°C, le résidu est repris dans 15 mL d'éthanol et 725 µL d'une solution 1N d'acide chlorhydrique sont ajoutés. Le milieu est agité 20 minutes à 50°C puis concentré sous vide. Le résidu obtenu est repris dans l'eau et extrait 3 fois au dichlorométhane ; les phases organiques sont réunies, lavées par une solution saturée de chlorure de sodium, séchées sur sulfate de magnésium anhydre et concentrées sous vide. Après purification par flash chromatographie sur gel de silice (dichlorométhane/ méthanol, gradient 95/5 à 8/2), 106 mg de l'énone sont obtenus avec un rendement de 12%.
LC/UV/MS (254nm) : T_{R} = 7,35 min, *m*/*z* = 400 [M+H]⁺
RMN-¹H (CDCl₃) : δ (ppm) 5,72 (s, 1H, 4-CH), 2,86 (m, 1H, 25-CH), 2,10-2,60 (massif, 3H, NCH₃), 0,70 (s, 3H, 18-CH₃).

### Etape 4: Synthèse de la 25-[méthyl(7-nitro-2,1,3-benzoxadiazol-4-yl)amino]-27-norcholest-5-èn-3-one

Dans un ballon, sont introduits 106 mg (0,265 mmol) de 25-(N-méthylamino)-27-norcholest-4-èn-3-one obtenu à l'étape 3, 58,3 mg (0,292 mmol) de 4-chloro-7-nitrobenzofurazane et 74 µL de triéthylamine dans 8 mL de dichlorométhane. La solution est agitée une nuit à température ambiante à l'abri de la lumière puis le milieu réactionnel est dilué dans du dichlorométhane. La solution obtenue est lavée par une solution 1N d'acide chlorhydrique (3 fois), séchée sur sulfate de magnésium anhydre et concentrée sous vide. Le résidu obtenu est purifié par flash chromatographie sur gel de silice (éluant dichlorométhane / acétate d'éthyle 95/5) puis par flash chromatographie en phase inverse (éluant acétonitrile). 27 mg du produit attendu sont obtenus sous forme de solide marron (rendement 18%).
LC/UV/MS (254 nm) : T_{R} = 4,75 min, *m*/*z* = 563 [M+H]⁺
RMN-¹H (CDCl₃) : δ (ppm) 8,44 (d, 1H, NBD-CH), 6,16 (d, 1H, NBD-CH), 5,71 (s, 1H, 4-CH), 2,93-3,25 (massif, 4H, 25-CH et NCH₃), 0,66 (d, 3H, 18-CH₃).

### Etape 4' : Synthèse de la 25-((N-(+)-biotinoyl-N-méthyl) amino)-27-norcholest-4-èn-3-one

Dans un ballon, sont introduits 82 mg (0,205 mmol) de 25-(N-méthylamino)-27-norcholest-4-èn-3-one obtenu à l'étape 3, 43 mg (0,226 mmol) de 1-(3-diméthylaminopropyl)-3-ethylcarboiimide, 33 mg (0,267 mmol) de N,N-diméthylaminopyridine et 55 mg (0,226 mmol) de D(+)-biotine dans 2 mL de N,N-diméthylformamide et 1 mL de dichlorométhane. Le milieu réactionnel est agité une nuit à température ambiante puis il est noyé dans de l'eau et extrait 3 fois au dichlorométhane. Les phases organiques sont réunies, lavées par une solution saturée de chlorure de sodium, séchées sur sulfate de magnésium anhydre et concentrées sous vide. Après purification par flash chromatographie sur gel de silice (gradient 100% dichlorométhane à 95/5 dichlorométhane / méthanol), 90 mg de l'amide sont obtenus avec un rendement de 70%.
LC/UV/MS (254 nm) : T_{R} = 9,14 min, *m*/*z* = 626 [M+H]⁺

### Exemple 1g - Synthèse de la 19-biotinyloxy-cholest-4-èn-3-one

Dans un ballon, sont introduits 112 mg (0,582 mmol) de 1-(3-diméthylaminopropyl)-3-éthylcarboiimide, 84 mg (0,688 mmol) de N,N-diméthylaminopyridine et 142 mg (0,582 mmol) de D(+)-biotine dans 3,5 mL de N,N-diméthylformamide et 2 mL de dichlorométhane. Le milieu est agité 45 minutes à température ambiante puis 212 mg (0,529 mmol) de 19-hydroxy-cholest-4-èn-3-one* en solution dans 2 mL de N,N-diméthylformamide sont ajoutés goutte-à-goutte. Le milieu est agité 48h à température ambiante puis il est concentré sous vide. Le résidu est repris dans de l'eau et extrait 3 fois à l'acétate d'éthyle. Les phases organiques sont réunies, lavées par une solution saturée de chlorure de sodium, séchées sur sulfate de magnésium anhydre et concentré sous vide. Après purification par flash chromatographie sur gel de silice (gradient 100% dichlorométhane à 95/5 dichlorométhane / méthanol), 180 mg de l'ester 19-biotinyloxy-cholest-4-èn-3-one sont obtenus avec un rendement de 54%.
RMN-¹H (CDCl₃) : δ (ppm) 5,91 (s, 1H), 4,72 (d, 1H), 4,52 (m, 1H), 4,32 (m, 1H), 4,12 (d, 1H), 3,13 (m, 1H), 2,93 (dd, 1H), 0,70 (s, 3H)
LC/UV/MS (254 nm) : T_{R} = 4,85 min, *m*/*z* = 627 [M+H]⁺
* le composé 19-hydroxy-cholest-4-èn-3-one est disponible commercialement.

### Exemple 1h - Synthèse de la 2-méthyl-cholest-4-èn-3-one

La 2-méthyl-cholest-4-èn-3-one a été préparée selon la méthode décrite dans l'article suivant : Julia, S. & al. Journal of Chemical society (1964), Aug, 2633-9.

### Exemple 1i - Synthèse de la 4-méthoxy-cholest-4-èn-3-one

La 4-méthoxy-cholest-4-èn-3-one a été préparée selon la méthode décrite dans les articles suivants : Patel, K. M. & al. Journal of Organic Chemistry (1975), 40(10), 1504-5 et Engelfried, O. & al Patent DE 1117112 (1961).

### Exemple 2 - Synthèse de composés de formule (I)

### Exemple 2a - Méthode générale A :

Dans un ballon, sont introduits un équivalent de cétone et six équivalents de chlorhydrate d'hydroxylamine dans de la pyridine (environ 10 à 20 mL/mmol). La solution est agitée pendant une nuit à température ambiante puis le milieu réactionnel est concentré sous vide. Le résidu obtenu est repris dans l'eau et extrait au dichlorométhane ou à l'acétate d'éthyle ; la phase organique est séparée, lavée à l'eau, séchée sur sulfate de magnésium anhydre et concentrée sous vide. Si nécessaire le produit est purifié par chromatographie flash sur gel de silice.

### Exemple 2b - Synthèse du 3-oxyimino-4-fluoro-cholest-4-ène

Le 3-oxyimino-4-fluoro-cholest-4-ène est obtenu à partir de la 4-fluoro-cholest-4-èn-3-one, préparée à l'exemple 1a, avec un rendement quantitatif selon la méthode A.
RMN-¹H (CDCl₃) : δ (ppm) 3,02 (dd, 2H, 2-CH₂), 2,23 (td, 1H, 6-CHₐ) 0,69 (3H, 18-CH₃).
RMN-¹⁹F (CDCl₃) : δ (ppm, non calibré) -137,35 (s, 4-CF)
LC/UV/MS (254 nm) : T_{R} = 6,42 min, *m*/*z* = 418 [M+H]⁺

### Exemple 2c - Synthèse du 3-oxyimino-6β-fluoro-cholest-4-ène

Le 3-oxyimino-6β-fluoro-cholest-4-ène est obtenu sous forme de mélange syn/anti à partir de la 6β-fluoro-cholest-4-èn-3-one, préparée à l'exemple 1b, avec un rendement de 93% selon la méthode A.
RMN-¹H (CDCl₃) : δ (ppm) 6,75 (d, 0,3H, 4-CH [syn]), 6,07 (d, 0.7H, 4-CH [anti]), 5,01 (dt, 0,3H, 6-CH [syn]), 4,97 (dt, 0,7H, 6-CH [anti]), 3,07 (d, 0,7H, 2-CHₐ [anti]), 0,72 (s, 3H, 18-CH₃).
RMN-¹⁹F (CDCl₃) : δ (ppm, non calibré) -159,23 (td, 6-CF [anti]), -162,05 (td, 6-CF [syn])
LC/UV/MS (254 nm) : T_{R} = 5,88 et 5,96 min (syn/anti), *m*/*z* = 418 [M+H]⁺

### Exemple 2d - Synthèse du 3-oxyimino-2,2-difluoro-cholest-4-ène

Le 3-oxyimino-2,2-difluoro-cholest-4-ène est obtenu sous forme de mélange syn/anti à partir de la 2,2-difluoro-4-cholestèn-3-one ; préparée à l'exemple 1c ; avec un rendement de 89% selon la méthode A.
RMN-¹H (CDCl₃) : δ (ppm) 7,18 (d, 0,3H, 4-CH [syn]), 6,49 (d, 0,7H, 4-CH [anti]), 3,09 (d large, 0,7H), 0,71 (s, 3H, 18-CH₃).
LC/UV/MS (254 nm) : T_{R} = 6,06 min, *m*/*z* = 436 [M+H]⁺

### Exemple 2e - Synthèse du 3-oxyimino-2,6-difluoro-cholest-4-ène

Le 3-oxyimino-2,6-difluoro-cholest-4-ène est obtenu sous forme de mélange syn/anti à partir de la 2,6-difluoro-cholest-4-èn-3-one, préparée à l'exemple 1c, avec un rendement de 97% selon la méthode A.
RMN-¹H (CDCl₃) : δ (ppm) 6,84 (s, 0,35H, 4-CH [syn]), 6,25 (s, 0,65H, 4-CH [anti]), 5,94 (d large, 1H, 2-CH), 5,13 (d large, 1H, 6-CH), 0,70 (s, 3H, 18-CH₃).
RMN-¹⁹F (CDCl₃) : δ (ppm, non calibré) -171,52 (td, 2-CF [syn]), -180,75 (td, 2-CF [anti]), -183,56 (d, 6-CF [syn]), -184,02 (d, 6-CF [anti]).
LC/UV/MS (254 nm) : T_{R} = 5,68 min, *m*/*z* = 436 [M+H]⁺

### Exemple 2f - Synthèse du 3-oxyimino-2α-fluoro-cholest-4-ène

Le 3-oxyimino-2α-fluoro-cholest-4-ène est obtenu sous forme de mélange syn/anti à partir de la 2α-fluoro-cholest-4-èn-3-one, préparée à l'exemple 1d, avec un rendement de 97% selon la méthode A.
RMN-¹H (CDCl₃) : δ (ppm) 6,87 (s, 0,3H, 4-CH [syn]), 6,21 (s, 0,7H, 4-CH [anti]), 5,08 (ddd, 1H, 2-CH), 3,08 (d large, 0,7H), 0,69 (s, 3H, 18-CH₃).
RMN-¹⁹F (CDCl₃) : δ (ppm, non calibré) -183,12 (ddd, 2-CF [syn]), -183,66 (ddd, 2-CF [anti])
LC/UV/MS (254 nm) : T_{R} = 5,78 et 5,99 min (syn/anti), m/z = 418 [M+H]⁺

### Exemple 2g - Synthèse du 3-oxyimino-25-fluoro-cholest-4-ène

Le 3-oxyimino-25-fluoro-cholest-4-ène est obtenu sous forme de mélange syn/anti à partir de la 25-fluoro-cholest-4-èn-3-one, préparée à l'exemple 1e, avec un rendement de 83% selon la méthode A.
RMN-¹H (CDCl₃) : δ (ppm) 6,46 (s, 0,4H, 4-CH [syn]), 5,77 (s, 0,6H, 4-CH [anti]), 3,04 (d, 0,6H, 2-CHₐ[anti]), 0,70 (s, 3H, 18-CH₃).
RMN-¹⁹F (CDCl₃) : δ (ppm, non calibré) -137,01 (sept, 25-CF).
LC/UV/MS (254 nm) : T_{R} = 4,44 et 4,84 min (syn/anti), m/z = 418 [M+H]⁺

### Exemple 2h - Synthèse du 3-oxyimino-25-[méthyl(7-nitro-2,1,3-benzoxadiazol-4-yl) amino]-27-norcholest-4-ène

Dans un ballon, sont introduits 25 mg (0,044 mmol) de 25-[méthyl(7-nitro-2,1,3-benzoxadiazol-4-yl) amino]-27-norcholest-4-èn-3-one, préparée à l'exemple 1f- étape 4, et 25 mg (0,36 mmol) de chlorhydrate d'hydroxylamine dans 2 mL de pyridine. La solution est agitée une nuit à température ambiante puis le milieu réactionnel est dilué dans du dichlorométhane. La solution obtenue est lavée à l'eau, séchée sur sulfate de magnésium anhydre et concentrée sous vide. 26 mg du 3-oxyimino-25-[méthyl(7-nitro-2,1,3-benzoxadiazol-4-yl) amino]-27-norcholest-4-ène sont ainsi obtenus sous forme de solide orange (rendement 89%).
LC/UV/MS (254 nm) : T_{R} = 3,34, 3,64 et 3,95 min, *m*/*z* = 578 [M+H]⁺
RMN-¹H (CDCl₃) : δ (ppm) 8,45 (d, 1H, NBD-CH), 6,45 (s, 0,34H, 4-CH [syn]), 6,16 (d, 1H, NBD-CH), 5,80 (s, 0,66H, 4-CH [anti]), 2,94-3,30 (massif, 4H, 25-CH et NCH₃), 0,64 (d, 3H, 18-CH₃).

### Exemple 2i - Synthèse du 3-oxyimino-25-((N-(+)-biotinoyl-N-méthyl) amino)-27-norcholest-4-ène

Dans un ballon, sont introduits 65 mg (0,104 mmol) de 25-((N-(+)-biotinoyl-N-méthyl) amino)-27-norcholest-4-èn-3-one, préparée à l'exemple 1f - étape 4', et 65 mg (0,935 mmol) de chlorhydrate d'hydroxylamine dans 2 mL de pyridine. La solution est agitée une nuit à température ambiante puis la pyridine est concentrée sous vide. Le résidu est repris dans de l'eau et extrait 4 fois à l'acétate d'éthyle. Les phases organiques sont réunies, lavées par une solution saturée de chlorure de sodium, séchées sur sulfate de magnésium anhydre et concentrées sous vide. 66 mg de le 3-oxyimino-25-((N-(+)-biotinoyl-N-méthyl) amino)-27-norcholest-4-ène sont ainsi obtenus sous forme de solide beige (rendement 98%).
LC/UV/MS (254 nm) : T_{R} = 8,56 et 8,81 min, *m*/*z* = 641 [M+H]⁺
RMN-¹H (CDCl₃) : δ (ppm) 6,46 (s, 0,3H, 4-CH [syn]), 5,77 (s, 0,7H, 4-CH [anti]), 2,68-2,81 (massif, 4H), 2,85-3,23 (massif, 3H), 3,82 (m, 0,5H), 4,33 (m, 1H), 4,51 (m, 1H), 4,73 (m, 0,5H), 4,90-5,01 (massif, 1H), 5,35-5,49 (massif, 1H), 0,68 (s large, 3H, 18-CH₃).

### Exemple 2j - 3-oxyimino-7α-hydroxy-cholest-4-ène

Le 3-oxyimino-7α-hydroxy-cholest-4-ène est obtenu sous forme de mélange syn/anti à partir de la 7α-hydroxy-cholest-4-èn-3-one* avec un rendement quantitatif selon la méthode A.
RMN-¹H (CDCl₃) : δ (ppm) 6,56 (s, 0.3H), 5,87 (s, 0,7H), 3,85-3,94 (massif, 1H), 3,92-4,08 (massif, 1H), 3,05 (dt, 0,7H), 2,56 (d large, 1H), 1,07 (s, 3H), 0,90 (d, 3H), 0,86 (dd, 6H), 0,69 (s, 3H)
LC/UV/MS (254 nm) : T_{R} = 3,19 et 3,56 min (syn/anti), *m*/*z* = 416 [M+H]⁺
* La 7α-hydroxy-4-cholestèn-3-one est commercialement disponible.

### Exemple 2k - 3-oxyimino-19-hydroxy-cholest-4-ène

Le 3-oxyimino-19-hydroxy-cholest-4-ène est obtenu sous forme de mélange syn/anti à partir de la 19-hydroxy-cholest-4-èn-3-one* avec un rendement de 96% selon la méthode A.
RMN-¹H (CDCl₃) : δ (ppm) 6,75 (s, 0,25H), 6,07 (s, 0,75H), 3,69-3,81 (massif, 1H), 3,92-4,08 (massif, 1H), 2,96 (d large, 1H), 0,68 (s, 3H)
LC/UV/MS (254 nm) : T_{R} = 3,81 et 4,27 min (syn/anti), m/z = 416 [M+H]⁺
* La 19-hydroxy-4-cholestèn-3-one est commercialement disponible.

### Exemple 21 - 3-oxyimino-19-biotinyloxy-cholest-4-ène

Le 3-oxyimino-19-biotinyloxy-cholest-4-ène est obtenu sous forme de mélange syn/anti à partir de l'ester 19-biotinyloxy-cholest-4-èn-3-one, préparée à l'exemple 1 g, avec un rendement de 92% selon la méthode A.
RMN-¹H (CDCl₃) : δ (ppm) 6,76 (s, 0.3H), 6,62 (s large, 1H), 5,93 (s, 0.7H), 4,89 (d, 0,7H), 4,73 (d, 0,3H), 4,50 (m, 1H), 4,34 (m, 1H), 4,03 (d, 0,3H), 3,85 (d, 0,7H), 2,15-3,20 (massif, 8H), 0,69 (s, 3H)
LC/UV/MS (254 nm) : T_{R} = 4,41 et 4,55 min (syn/anti), *m*/*z* = 642 [M+H]⁺

### Exemple 2m - Synthèse du 3-oxyimino-2-méthyl-cholest-4-ène

Le 3-oxyimino-2-méthyl-cholest-4-ène est obtenu à partir de la 2-méthyl-cholest-4-èn-3-one, préparée à l'exemple 1 h, avec un rendement quantitatif selon la méthode A.
RMN-¹H (CDCl₃) : δ (ppm) 6,44 (s, 0,7H), 5,78 (s, 0,20H), 0,70 (s, 3H)
LC/UV/MS (254 nm) : T_{R} = 6,42 et 6,74 min (syn/anti), *m*/*z* = 414 [M+H]⁺

### Exemple 2n - Synthèse du 3-oxyimino-4-méthoxy-cholest-4-ène

Le 3-oxyimino-4-méthoxy-cholest-4-ène est obtenu à partir de la 4-méthoxy-cholest-4-èn-3-one, préparée à l'exemple 1i, avec un rendement quantitatif selon la méthode A.
RMN-¹H (CDCl₃) : δ (ppm) 3,57 (s, 3H), 3,03 (dd, 2H), 0,69 (s, 3H)
LC/UV/MS (254 nm) : T_{R} = 6,36 min, *m*/*z* = 430 [M+H]⁺

### ETUDE PHARMACOLOGIQUE

Les composés ont été testés selon les protocoles suivants :

### Effets des composés de formules (I) sur la survie des motoneurones

Pour mettre en évidence l'action neuroprotectrice des composés de formules (I), la demanderesse a étudié leur activité sur un modèle *in vitro* de privation trophique de motoneurones de rats. On pourra se référer utilement à la demande de brevet WO 0142784 de la demanderesse sur la mise en culture des motoneurones de moelle épinière.

La moelle épinière d'embryons E14 de rat est disséquée et la partie ventrale est dissociée par trituration après trypsination.

Les motoneurones sont séparés des autres cellules spinales par une méthode connue (Camu et col, 1993, Purification of spinal motoneurons from chicken and rat embryos by immunopanning. In " Immunoselection Strategies for Neural cell culture ", Neuroprotocols : A companion to Methods in Neurosciences 2, 191-199 ; Henderson et col., 1993, Neutrophins promote motor neuron survival and are present in embryonic limb bud. Nature 363 (6426) :266-70).

Les cellules sont centrifugées sur un gradient de densité. Les motoneurones sont enrichis dans la fraction des grandes cellules (les moins denses). Les cellules de cette fraction sont incubées avec un anticorps anti-p75, un antigène de surface présent sur les motoneurones.

Des anticorps secondaires couplés à des billes magnétiques sont ajoutés et le mélange de cellules est passé à travers une colonne dans un aimant (Arce et al, 1999 Cardiotrophin-1 requires LIFRbeta to promote survival of mouse motoneurons purified by a novel technique. J. Neurosci Res 55(1) : 119-26). Seuls les motoneurones sont retenus : leur pureté est de l'ordre de 90%.

Les motoneurones sont ensemencés à faible densité dans des puits de culture sur un substrat de polyornithine-laminine dans un milieu neurobasal (GIBCO) supplémenté selon Raoul et col, 1999, Programmed cell death of embryonic motoneurons triggered through the Fas death receptor. J Cell Biol 147(5) :1049-62.

Des contrôles négatifs (absence de facteurs trophiques) et positifs (en présence de BDNF (Brain-Derived Neurotrophic Factor) à 1 ng/ml, GDNF (Glial-Derived Neurotrophic Factor) à 1 ng/ml et CNTF (Ciliary Neurotrophic Factor) à 10 ng/ml, commercialisés par la société américaine PEPROTECH, Inc. et la société Sigma-Aldrich, sont inclus dans chaque série.

Les composés à tester sont ajoutés 60 minutes après l'ensemencement et les cultures sont maintenues à 37°C sous 5% de CO₂ pendant 3 jours.

Les motoneurones ont une tendance spontanée à mourir en l'absence de facteurs neurotrophiques (Pettmann et Henderson, 1998, Neuronal cell death. Neuron 20(4):633-47). Après 3 jours, la survie est évaluée par une mesure de fluorescence après incubation des cellules en présence de calcéine qui devient fluorescente dans les cellules vivantes.

Après 3 jours en culture à 37°C, sous 5% de CO₂ et en humidité saturante, jusqu'à 50% des motoneurones ensemencés initialement survivent dans le milieu supplémenté en facteurs neurotrophiques, alors que moins de 15% des motoneurones survivent en milieu de culture non additionné de facteurs neurotrophiques..

L'activité neuroprotectrice des composés à tester a été évaluée par leur capacité à empêcher la mort des motoneurones quand ils sont ajoutés au milieu Neurobasal (GIBCO) en comparaison avec la survie des motoneurones en milieu additionné de facteurs neurotrophiques.

Les composés de formule I selon l'invention ont montré une activité neuroprotectrice à une concentration capable de permettre un meilleur taux de survie des motoneurones dans le milieu Neurobasal.

Ce taux de survie est exprimé par le nombre de cellules vivantes après traitement avec le composé à tester par rapport à la survie induite par les facteurs neurotrophiques. Ce rapport peut représenter donc le pourcentage de survie due au composé testé par rapport à la survie induite par les facteurs neurotrophiques. Si le rapport est supérieur à 0, l'effet des composés est positif sur la survie des motoneurones.

Les résultats obtenus sont les suivants :

| Composé de l'exemple | Concentration en µM | Ratio |
|---|---|---|
| 2b | 3 | 0,73 |
| 2c | 3 | 0,50 |
| 2d | 3 | 0,50 |
| 2e | 3 | 0,37 |
| 2f | 3 | 0,27 |
| 2h | 1 | 0,66 |
| 2i | 3 | 0,42 |
| 2k | 3 | 0,49 |
| 2l | 1 | 0,32 |
| 2m | 1 | 0,42 |
| 2n | 1 | 0,34 |

De par leur effet trophique sur les motoneurones spinaux, les composés de formule (I) selon l'invention se montrent donc potentiellement utiles comme médicament, notamment dans le traitement des amyotrophies, en particulier dans le traitement de la sclérose latérale amyotrophique ou des amyotrophies spinales infantiles, et dans le traitement des traumatismes de la moelle épinière.

## Revendications

1. Composé, **caractérisé en ce qu'**il s'agit du :
3-oxyimino-4-fluoro-cholest-4-ène ;
3-oxyimino-6β-fluoro-cholest-4-ène ;
3-oxyimino-2,2-difluoro-cholest-4-ène ;
3-oxyimino-2,6-difluoro-cholest-4-ène ;
3-oxyimino-2α-fluoro-cholest-4-ène ;
3-oxyimino-25-[méthyl(7-nitro-2,1,3-benzoxadiazol-4-yl)amino]-27-norcholest-4-ène ;
3-oxyimino-25-((N-(+)-biotinoyl-N-méthyl) amino)-27-norcholest-4-ène ;
3-oxyimino-19-hydroxy-cholest-4-ène ;
3-oxyimino-19-biotinyloxy-cholest-4-ène ;
3-oxyimino-2-méthyl-cholest-4-ène ;
3-oxyimino-4-méthoxy-cholest-4-ène.

2. Composé tel que décrit à la revendication 1 pour son utilisation comme médicament.

3. Composé tel que décrit à la revendication 1 pour son utilisation comme médicament cytoprotecteur, préférentiellement cardioprotecteur et/ou neuroprotecteur et/ou hépatoprotecteur.

4. Composé tel que décrit à la revendication 1 pour son utilisation dans le traitement ou à la prévention de la nécrose et/ou de l'apoptose pathologique et/ou de la nécroptose.

5. Composé tel que décrit à la revendication 1 pour son utilisation dans le traitement des pathologies ou traumatismes liés à la dégénérescence ou à la mort des cellules, particulièrement des cellules cardiaques et/ou des neurones, que celles-ci soient naturelles ou accidentelles.

6. Composé tel que décrit à la revendication 1 pour son utilisation dans le traitement des affections comme :
▪ les maladies des os, des articulations, du tissu conjonctif et du cartilage ;
▪ les maladies musculaires telles que la dystrophie musculaire ;
▪ les maladies de la peau ;
▪ les maladies cardiovasculaires ;
▪ les maladies circulatoires ;
▪ les maladies hématologiques et vasculaires ;
▪ les maladies du poumon dont les maladies chroniques obstructives des poumons;
▪ les maladies du tractus gastro-intestinal ;
▪ les maladies du foie ;
▪ les maladies du pancréas ;
▪ les maladies métaboliques ;
▪ les maladies des reins ;
▪ les infections virales et bactériennes ;
▪ les intoxications sévères ;
▪ les affections dégénératives associées au Syndrome Immuno Déficitaire Acquis (SIDA) ;
▪ les désordres associés au vieillissement ;
▪ les maladies inflammatoires ;
▪ les maladies auto-immunes ;
▪ les désordres dentaires ;
▪ les maladies ou désordres ophtalmiques ;
▪ les désordres des voies auditives ;
▪ les maladies associées aux mitochondries;
▪ les maladies neurologiques et neurodégénératives.

7. Composé tel que décrit à la revendication 1 pour son utilisation dans le traitement des maladies neurodégénératives.

8. Composé selon la revendication 6 ou 7, **caractérisée en ce qu'**il est utilisé dans le traitement d'une maladie neurodégénérative choisie parmi la maladie de Huntington, les maladies chroniques neurodégénératives, héréditaires ou sporadiques, les lésions neuronales liées au vieillissement, les neuropathies périphériques héréditaires ou lésionnelles, la maladie de Charcot-Marie-Tooth, les neuropathies diabétiques ou induites par les traitement anticancéreux, les épilepsies, les maladies chroniques démyélinisantes et neurodégénératives, notamment la sclérose en plaque et les leucodystrophies , les traumatismes du cerveau, des nerfs périphériques ou de la moelle épinière, les ischémies du cerveau ou de la moelle épinière, les dégénérescences héréditaires, lésionnelles ou liées au vieillissement des neurones sensoriels de la vision ou les dégénérescences du nerf optique, les dégénérescences héréditaires, traumatiques ou liées au vieillissement des neurones sensoriels de l'ouïe, les atrophies lobaires et les démences vasculaires, les maladies et traumatismes liés à la dégénérescence des motoneurones, les amyotrophies spinales, particulièrement infantiles, la sclérose en plaques, les scléroses latérales amyotrophiques et les traumatismes de la moelle épinière ou des nerfs moteurs périphériques.

9. Composé selon l'une quelconque des revendications 6 à 8, **caractérisée en ce qu'**il est utilisé dans le traitement des amyotrophies spinales, particulièrement infantiles, ou des scléroses latérales amyotrophiques.

10. 3-oxyimino-25-[méthyl(7-nitro-2,1,3-benzoxadiazol-4-yl)amino]-27-norcholest-4-ène, du 3-oxyimino-25-((N-(+)-biotinoyl-N-méthyl) amino)-27-norcholest-4-ène ou du 3-oxyimino-19-biotinyloxy-cholest-4-ène, pour leur utilisation en tant que traceur ou agent de marquage.

11. Composition pharmaceutique, **caractérisée en ce qu'**elle renferme à titre de principe actif au moins un composé tel que décrit à la revendication 1 ainsi qu'un excipient pharmaceutiquement acceptable.

## Patentansprüche

1. Verbindung, **dadurch gekennzeichnet, dass** es sich um Folgendes handelt:
3-Oxyimino-4-fluor-cholest-4-en;
3-Oxyimino-6β-fluor-cholest-4-en;
3-Oxyimino-2,2-difluor-cholest-4-en;
3-Oxyimino-2,6-difluor-cholest-4-en;
3-Oxyimino-2α-fluor-cholest-4-en;
3-Oxyimino-25-[methyl(7-nitro-2,1,3-benzoxadiazol-4-yl)amino]-27-norcholest-4-en;
3-Oxyimino-25-((N-(+)-biotinoyl-N-methyl)amino)-27-norcholest-4-en;
3-Oxyimino-19-hydroxy-cholest-4-en;
3-Oxyimino-19-biotinyloxy-cholest-4-en;
3-Oxyimino-2-methyl-cholest-4-en;
3-Oxyimino-4-methoxy-cholest-4-en.

2. Verbindung nach Anspruch 1 zur Verwendung als Medikament.

3. Verbindung nach Anspruch 1 zur Verwendung als zellschützendes Medikament, vorzugsweise als herzschützendes und/oder nervenschützendes und/oder leberschützendes Medikament.

4. Verbindung nach Anspruch 1 zur Verwendung bei der Behandlung oder Verhütung von Nekrose und/oder pathologischer Apoptose und/oder Nekroptose.

5. Verbindung nach Anspruch 1 zur Verwendung bei der Behandlung von Pathologien oder Traumata in Verbindung mit der Degeneration oder dem Absterben von Zellen, insbesondere von Herzzellen und/oder Neuronen, unabhängig davon ob natürlich oder unbeabsichtigt.

6. Verbindung nach Anspruch 1 zur Verwendung bei der Behandlung von Erkrankungen wie:
▪ Krankheiten von Knochen, Gelenken, Bindegewebe und Knorpel;
▪ Muskelkrankheiten wie Muskeldystrophie;
▪ Krankheiten der Haut;
▪ kardiovaskuläre Krankheiten;
▪ Kreislaufkrankheiten;
▪ hämatologische und vaskuläre Krankheiten;
▪ Lungenkrankheiten, darunter chronische obstruktive Lungenkrankheiten;
▪ Krankheiten des Magen-Darm-Trakts;
▪ Krankheiten der Leber;
▪ Krankheiten der Bauchspeicheldrüse;
▪ Stoffwechselkrankheiten;
▪ Nierenkrankheiten;
▪ Virus- und Bakterieninfektionen;
▪ ernsthafte Vergiftungen;
▪ degenerative Störungen in Verbindung mit AIDS (erworbenes Immunschwächesyndrom);
▪ mit dem Altern assoziierte Störungen;
▪ Entzündungskrankheiten;
▪ Autoimmunkrankheiten;
▪ dentale Störungen;
▪ ophthalmische Krankheiten oder Störungen;
▪ Störungen der Hörbahnen;
▪ mit den Mitochondrien assoziierte Krankheiten;
▪ neurologische und neurodegenerative Krankheiten.

7. Verbindung nach Anspruch 1 zur Verwendung bei der Behandlung von neurodegenerativen Krankheiten.

8. Verbindung nach Anspruch 6 oder 7, **dadurch gekennzeichnet, dass** sie bei der Behandlung einer neurodegenerativen Krankheit benutzt wird, ausgewählt aus der Huntington-Krankheit, chronischen neurodegenerativen Krankheiten, erblich oder sporadisch, neuronalen Verletzungen in Verbindung mit dem Altern, erblichen oder läsionalen peripheren Neuropathien, Charcot-Marie-Tooth-Krankheit, diabetischen Neuropathien oder durch Antikrebsmittel verursachten Neuropathien, Epilepsien, chronischen demyelinisierenden und neurodegenerativen Krankheiten, insbesondere Multiple Sklerose und Leukodystrophien, Traumata des Gehirns, der peripheren Nerven oder des Rückenmarks, Ischämien von Gehirn oder Rückenmark, erblichen, läsionalen oder mit dem Altern verbundenen Degenerationen von sensorischen Neuronen des Sehens oder Degenerationen des Sehnervs, erblichen, traumatischen oder mit dem Altern verbundenen Degenerationen von sensorischen Neuronen des Hörens, Lappenatrophien und vaskulärer Demenz, Krankheiten und Traumata in Verbindung mit der Degeneration von Motoneuronen, spinalen Muskelatrophien, insbesondere infantilen, Multiple Sklerose, amyotropher Lateralsklerose und Traumata des Rückenmarks oder der peripheren motorischen Nerven.

9. Verbindung nach einem der Ansprüche 6 bis 8, **dadurch gekennzeichnet, dass** sie bei der Behandlung von spinalen Muskelatrophien, insbesondere infantilen, oder amyotropher Lateralsklerose benutzt wird.

10. 3-Oxyimino-25-[methyl(7-nitro-2,1,3-benzoxadiazol-4-yl)amino]-27-norcholest-4-en, 3-Oxyimino-25-((N-(+)-biotinoyl-N-methyl) amino)-27-norcholest-4-en oder 3-Oxyimino-19-biotinyloxy-cholest-4-en zur Verwendung als Tracer oder Markierungsmittel.

11. Pharmazeutische Zusammensetzung, **dadurch gekennzeichnet, dass** sie als Wirkstoff wenigstens eine Verbindung nach Anspruch 1 sowie einen pharmazeutisch akzeptablen Hilfsstoff enthält.

## Claims

1. Compound, **characterized in that** it is:
3-oxyimino-4-fluoro-cholest-4-ene;
3-oxyimino-6β-fluoro-cholest-4-ene;
3-oxyimino-2,2-difluoro-cholest-4-ene;
3-oxyimino-2,6-difluoro-cholest-4-ene;
3-oxyimino-2α-fluoro-cholest-4-ene;
3-oxyimino-25-[methyl(7-nitro-2,1,3-benzoxadiazol-4-yl)amino]-27-norcholest-4-ene;
3-oxyimino-25-((N-(+)-biotinoyl-N-methyl) amino)-27-norcholest-4-ene;
3-oxyimino-19-hydroxy-cholest-4-ene;
3-oxyimino-19-biotinyloxy-cholest-4-ene;
3-oxyimino-2-methyl-cholest-4-ene;
3-oxyimino-4-methoxy-cholest-4-ene.

2. Compound as described in claim 1 for use thereof as a medicament.

3. Compound as described in claim 1 for use thereof as a cytoprotective, preferentially cardioprotective and/or neuroprotective and/or hepatoprotective, medicament.

4. Compound as described in claim 1 for use thereof in the treatment or the prevention of necrosis and/or of pathological apoptosis and/or of necroptosis.

5. Compound as described in claim 1 for use thereof in the treatment of pathologies or traumas linked to the degeneration or death of cells, particularly cardiac cells and/or neurons, whether natural or accidental.

6. Compound as described in claim 1 for use thereof in the treatment of medical conditions such as:
▪ diseases of the bones, joints, connective tissue and cartilage;
▪ muscle diseases such as muscular dystrophy;
▪ skin diseases;
▪ cardiovascular diseases;
▪ circulatory diseases;
▪ haematological and vascular diseases;
▪ lung diseases including chronic obstructive lung diseases;
▪ diseases of the gastro-intestinal tract;
▪ liver diseases;
▪ diseases of the pancreas;
▪ metabolic diseases;
▪ kidney diseases;
▪ viral and bacterial infections;
▪ severe poisoning;
▪ degenerative disorders associated with Acquired Immune Deficiency Syndrome (AIDS);
▪ disorders associated with ageing;
▪ inflammatory diseases;
▪ autoimmune diseases;
▪ dental disorders;
▪ ophthalmic diseases or disorders;
▪ disorders of the auditory pathways;
▪ mitochondrial diseases;
▪ neurological and neurodegenerative diseases.

7. Compound as described in claim 1 for use thereof in the treatment of neurodegenerative diseases.

8. Compound according to claim 6 or 7, **characterized in that** it is used in the treatment of a neurodegenerative disease selected from Huntington's disease, hereditary or sporadic chronic neurodegenerative diseases, neuronal lesions related to ageing, peripheral neuropathies which are hereditary or due to a lesion, Charcot-Marie-Tooth disease, diabetic neuropathies or neuropathies induced by anticancer treatments, epilepsy, demyelinating and neurodegenerative chronic diseases, in particular multiple sclerosis and leukodystrophy, brain, peripheral nerve or spinal cord injuries, ischaemia of the brain or of the spinal cord, degeneration of the vision sensory neurons which is hereditary, due to a lesion or related to ageing, or degeneration of the optic nerve, degeneration of the hearing sensory neurons which is hereditary, due to trauma or related to ageing, lobar atrophy and vascular dementia, diseases and traumas linked to motor neuron degeneration, spinal muscular atrophies, particularly infantile, multiple sclerosis, amyotrophic lateral sclerosis and spinal cord or peripheral motor nerve injuries.

9. Compound according to any one of claims 6 to 8, **characterized in that** it is used in the treatment of spinal muscular atrophies, particularly infantile, or of amyotrophic lateral sclerosis.

10. 3-oxyimino-25-[methyl(7-nitro-2,1,3-benzoxadiazol-4-yl)amino]-27-norcholest-4-ene, 3-oxyimino-25-((N-(+)-biotinoyl-N-methyl) amino)-27-norcholest-4-ene or 3-oxyimino-19-biotinyloxy-cholest-4-ene, for use thereof as tracer or marking agent.

11. Pharmaceutical composition, **characterized in that** it contains as active ingredient at least one compound as described in claim 1 as well as a pharmaceutically acceptable excipient.
